(19) Europäisches Patentamt
European Patent Office
Office européen des brevets

(11) Numéro de publication : **0 321 353 B1**

(12) # FASCICULE DE BREVET EUROPEEN

(45) Date de publication du fascicule du brevet : **20.05.92 Bulletin 92/21**

(21) Numéro de dépôt : **88403210.3**

(22) Date de dépôt : **16.12.88**

(51) Int. Cl.$^5$ : **C07D 498/22,** C07D 471/22, G01N 33/533, // (C07D498/22, 273:00, 273:00, 221:00, 221:00), (C07D471/22, 259:00, 259:00, 221:00, 221:00, 221:00, 221:00, 221:00, 221:00)

(54) **Cryptates de terres rares, procédés d'obtention, intermédiaires de synthèse et application à titre de marqueurs fluorescents.**

(30) Priorité : **18.12.87 FR 8717765**

(43) Date de publication de la demande : **21.06.89 Bulletin 89/25**

(45) Mention de la délivrance du brevet : **20.05.92 Bulletin 92/21**

(84) Etats contractants désignés : **AT BE CH DE ES FR GB GR IT LI LU NL SE**

(56) Documents cités : **EP-A- 0 180 492**

(73) Titulaire : **CIS BIO INTERNATIONAL**
**RN 306**
**F-91000 Saclay (FR)**

(72) Inventeur : **Lehn, Jean-Marie**
**21, rue d'Oslo**
**F-67000 Strasbourg (FR)**
Inventeur : **Mathis, Gérard**
**17, Impasse de la Capelle des Ladres**
**F-30200 Bagnols-sur-Ceze (FR)**
Inventeur : **Alpha, Béatrice**
**2, rue Charles Appell**
**F-67000 Strasbourg (FR)**
Inventeur : **Deschenaux, Robert**
**53, rue de Zurich**
**F-67000 Strasbourg (FR)**
Inventeur : **Jolu, Etienne**
**Les Cyprès - 2, allée du Romarin**
**F-30200 Bagnols-sur-Ceze (FR)**

(74) Mandataire : **Gillard, Marie-Louise et al**
**Cabinet Beau de Loménie 55, Rue d'Amsterdam**
**F-75008 Paris (FR)**

EP 0 321 353 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention, qui résulte des travaux effectués en collaboration avec le Professeur J. M. LEHN et son équipe de recherche de l'université Louis Pasteur de Strasbourg, concerne le domaine de la fluorescence et plus particuliérement une nouvelle famille de complexes macropolycycliques de terres rares ou cryptates de terres rares, qui conviennent comme marqueurs fluorescents, notamment dans les dosages immunologiques.

Dans la demande de brevet EP-A-0180492 sont décrits des complexes macropolycycliques de terres rares qui sont constitués d'au moins un sel de terre rare complexé par un composé macropolycyclique de formule générale :

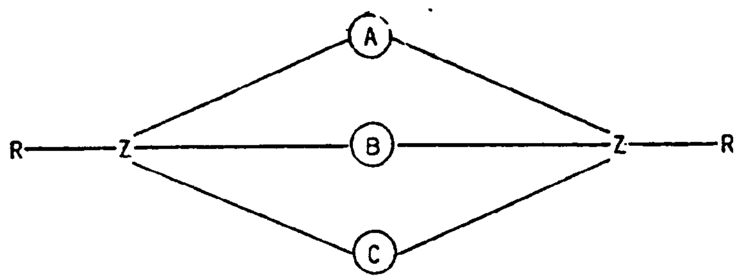

I

dans laquelle Z est un atome ayant 3 ou 4 valences, R est rien ou représente l'hydrogène, le groupe hydroxy, un groupe amino ou un radical hydrocarboné, les radicaux bivalents sont Ⓐ, Ⓑ et Ⓒ sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ, Ⓑ et Ⓒ comportant de plus au moins un motif moléculaire ou étant essentiellement constitué par un motif moléculaire, ledit motif moléculaire possédant une énergie de triplet supérieure à celle du niveau émissif de l'ion de terre rare complexé.

Ces cryptates conviennent notamment à titre de marqueurs de produits biologiques dans les méthodes de détection et de détermination immunologique par fluorescence.

Lorsque ces cryptates de terres rares sont utilisés pour marquer spécifiquement des molécules biologiques à l'aide d'une liaison covalente, ils peuvent être substitués sur l'un ou plusieurs de leurs motifs moléculaires constitutifs par un ou plusieurs substituants suffisamment accessibles et possédant un ou plusieurs motifs moléculaires permettant le couplage covalent avec la molécule biologique dans des conditions opératoires compatibles avec sa réactivité biologique.

Parmi ces motifs moléculaires, on peut citer, à titre d'exemples non limitatifs, les radicaux alkylamino, arylamino, isothiocyano, cyano, isocyano, thiocyano, carboxyle, hydroxyle, mercapto, phénol, imidazole, aldéhyde, époxyde, halogénure, thionyle, sulfonyle, nitrobenzoyle, carbonyle, triazo, succinimido, anhydride, halogénoacétate, hydrazino, dihalogénotriazinyle, etc. (Biol. Chem. 245, 3059 (1970)). La longueur du bras liant le cryptate à la molécule d'intérêt biologique peut varier par exemple de 1 à 20 atomes et contenir des atomes de carbone mais aussi des hétéroatomes tels que N, O, S, P.

On a maintenant trouvé deux familles de cryptates de terres rares fonctionnalisés qui peuvent être utilisés comme marqueurs fluorescents de molécules biologiques.

L'invention concerne également les procédés pour l'obtention de ces cryptates ainsi que les composés intermédiaires de synthèse.

Enfin, elle concerne aussi l'application de ces cryptates de terres rares comme marqueurs fluorescents de substances biologiques.

Les cryptates de terres rares fonctionnalisés selon la présente invention sont constitués d'au moins un sel de terre rare complexé par un composé macropolycyclique répondant à l'une des formules I ou II ci-après :

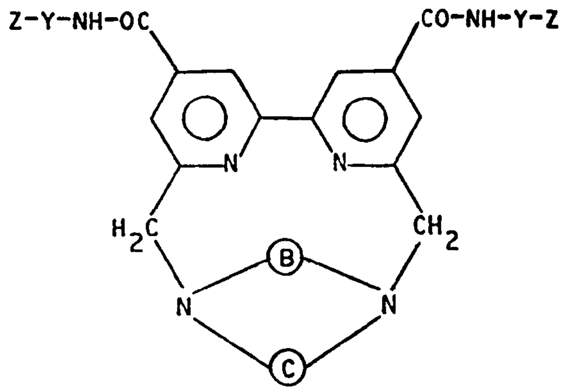

I

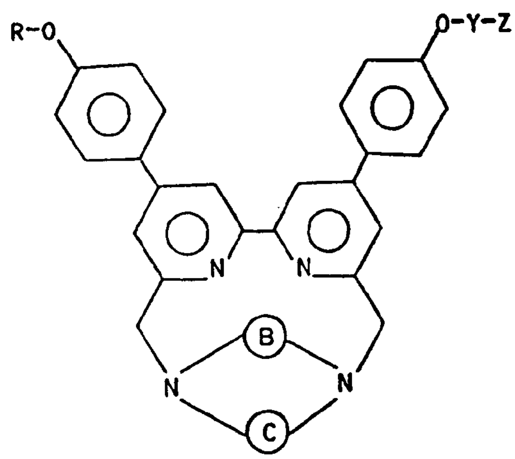

II

dans lesquels :

– le cycle de formule

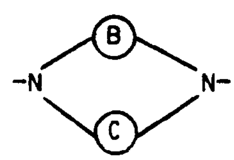

est l'un des cycles suivants :

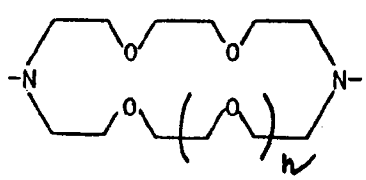

n= 0 ou 1

macrocycle [$N_2O_4$] ou cycle (22)

macrocycle [$N_2O_3$] ou cycle (21)

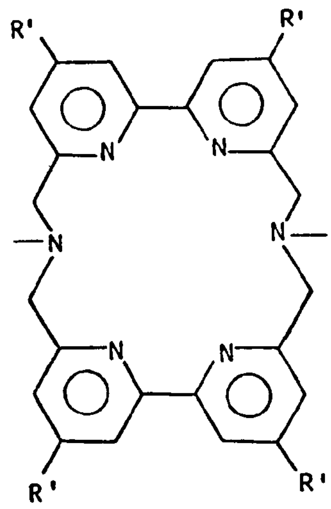

macrocycle bis-bipyridine

– Y est un groupe ou un bras d'espacement qui est constitué par un radical organique bivalent, choisi parmi les groupes alkylène linéaires ou ramifiées en $C_1$ à $C_{20}$, contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$-$C_8$ ou parmi les groupes arylène en $C_6$ à $C_{14}$, lesdits groupes alkylène, cycloalkylène ou arylène étant éventuellement substitués par des groupes alkyle, aryle ou sulfonate.

– Z est un groupe fonctionnel susceptible de se lier de façon covalente avec une substance biologique ;

– R est un groupe méthyle ou représente le groupe -Y-Z ;

– R′ est l'hydrogène ou un groupe -COOR″ dans lequel R″ est un groupe alkyle en $C_1$ à $C_{10}$ et représente de préférence le groupe méthyle, éthyle ou tertiobutyle ou bien R′ est un groupe-CO-NH-Y-Z.

Dans la présente description, on désigne par "groupe fonctionnel susceptible de se lier de façon covalente avec une substance biologique" tout groupe fonctionnel capable de se lier par liaison covalente, directement ou après activation, avec au moins l'une des fonctions naturellement présentes ou artificiellement introduites sur ladite substance biologique. De telles fonctions sont notamment les fonctions $NH_2$, COOH, SH ou OH. De tels groupes ainsi que les procédés d'activation sont décrits en détail par P. TIJSSEN dans "Practice and Theory of Enzyme immunossays" Elsevier 1985,document incorporé dans la présente description à titre de référence.

A titre d'exemples de groupes fonctionnels appropriés aux fins de l'invention, on peut citer notamment les groupes amino, thio, cyano, isocyano, isothiocyano, thiocyano, carboxyle, hydroxyle, maléimido, succinimido, mercapto, phénol, imidazole, aldéhyde, époxyde, halogénure, thionyle, sulfonyle, nitrobenzoyle, carbonyl, triazo, anhydride, halogénoacétate, hydrazino, acridine etc.

Les groupes particulièrement préférés sont les groupes amino, thio et carboxy qui doivent être activés avant le couplage covalent avec la substance biologique ainsi que les groupes maléimido, succinimido et isothiocyanate, lesquels peuvent se lier directement avec la substance biologique.

D'une manière générale, les cryptates selon l'invention peuvent être obtenus soit :

– par condensation du cycle

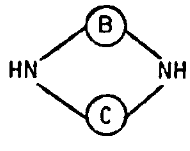

avec une dihalogénométhyl-6,6′, bipyridine-2,2′, disubstituée en 4,4′, substitution ultérieure par le groupe -NH-Y-Z ou le groupe -Y-Z et complexation du composé macropolycyclique obtenue par un sel de terre rare, ou

– par condensation d'une molécule de diaminométhyl-6,6′ bipyridine 2,2′ disubstituée en 4,4′, avec deux molécules de dihalogénométhyl-6,6′ bipyridine 2,2′ disubstitué en 4,4′, substitution ultérieure par le groupe -NH-Y-Z ou le groupe -Y-Z et complexation du composé macropolycyclique obtenu par un sel de terre rare.

On notera que dans les procédés ci-dessus, la complexation par un sel de terre rare peut avoir lieu avant l'étape de substitution. Cette complexation est réalisée de préférence avant l'étape de substitution.

Les différents procédés d'obtention des cryptates selon l'invention vont être maintenant décrits en détail.

PROCEDE A :

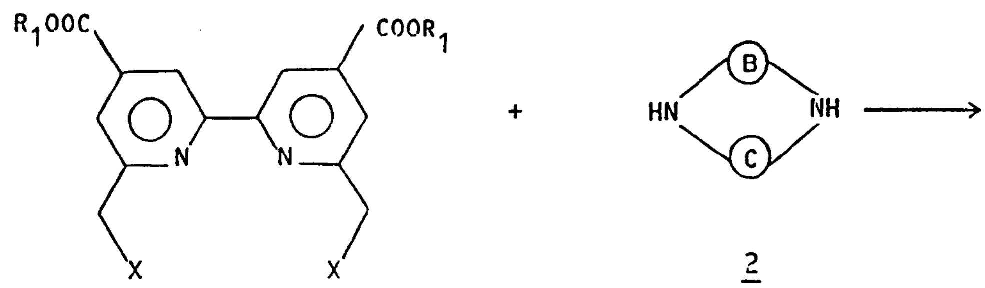

2

1

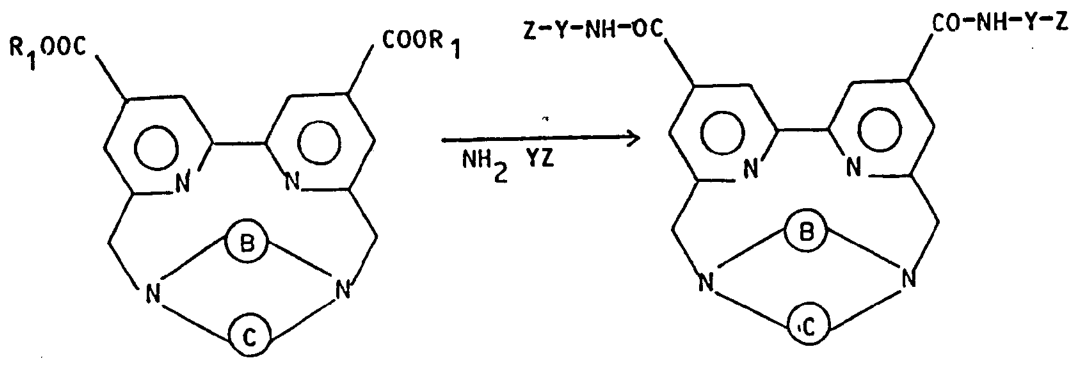


3

4

où X est un groupe halogéno et $R_1$ est un groupe alkyle ayant de 1 à 10 atomes de carbone et représente de préférence le groupe méthyle, éthyle ou tertiobutyle.

Selon ce procédé, on condense le dérivé halogéné de bipyridine 1 avec le macrocycle 2.

Cette réaction est avantageusement réalisée dans un solvant organique anhydre, tel que par exemple l'acétonitrile, en présence d'une base, telle qu'un carbonate d'ion alcalin, par exemple le carbonate de sodium ou le carbonate de lithium à la température de reflux du solvant. Le macrobicycle 3 obtenu est alors sous la forme d'un cryptate de l'ion alcalin utilisé par exemple sous la forme d'un cryptate de sodium.

On procède ensuite à l'aminolyse du composé macrobicyclique 3 par réaction de celui-ci avec une amine de formule $H_2N$-Y-Z dans laquelle les groupes Y et Z sont tels que définis précédemment, le groupe fonctionnel Z ayant été éventuellement bloqué par des moyens classiques. On opère avantageusement sous atmosphère d'azote et à la température ambiante. Lorsque la réaction est terminée, l'excès d'amine est éliminée par des moyens appropriés et le cryptate de formule 4 est récupéré selon des moyens classiques.

Le composé ainsi obtenu est ensuite transformé en un cryptate de terre rare par échange d'ions en chauffant au reflux une solution du cryptate d'ion alcalin du macrobicycle 4 dans du méthanol, en présence éventuellement de chloroforme, avec une solution d'un halogénure de terre rare dans du méthanol.

Comme indiqué précédemment, le cryptate d'ion alcalin 3 peut être, préalablement à l'aminolyse, transformé en un cryptate de terre rare par échange d'ions selon le mode opératoire décrit ci-dessus.

Le composé macrobicyclique 3 est un intermédiaire-clé pour la synthèse des cryptates de l'invention. Cet intermédiaire peut se présenter sous la forme d'un cryptate d'ion alcalin ou d'un cryptate de terre rare. Il constitue un autre objet de l'invention. Les intermédiaires particulièrement préférés aux fins de l'invention sont les composés de formule 3 dans lesquels $R_1$ est le groupe $CH_3$, $C_2H_5$ ou t-$C_4H_9$.

Selon une variante de mise en oeuvre de ce procédé, le cryptate d'ion alcalin peut être décomplexé en transformant d'abord celui-ci en le cryptate d'argent correspondant par action d'un excès de nitrate d'argent,

puis en traitant ce dernier par $H_2S$ [Helvetica Chimica Acta, vol. 67 (1984) 2264-2269]. On obtient alors le composé macropolycyclique 3 sous la forme du cryptand libre qui constitue également un intermédiaire de synthèse, lequel peut être ensuite complexé, par des moyens classiques en un cryptate de terre rare.

On notera que les cryptates de formules (I) ou (II) dans lesquelles Z est autre qu'un groupe amino peuvent être obtenus à partir des cryptates de formules (I) ou (II) dans lesquelles Z est le groupe amino par des procédés classiques bien connus de l'homme de l'art.

PROCEDE B :

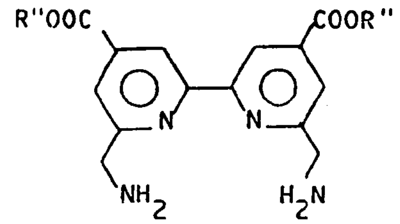

5

+ 2

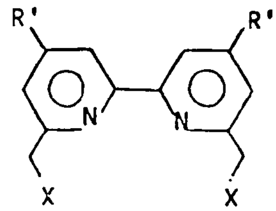

6

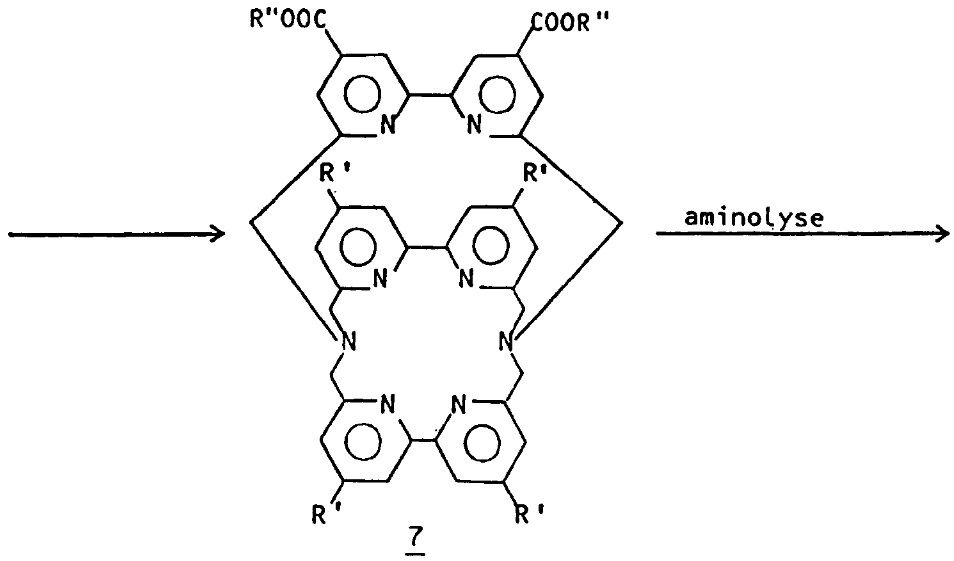


7

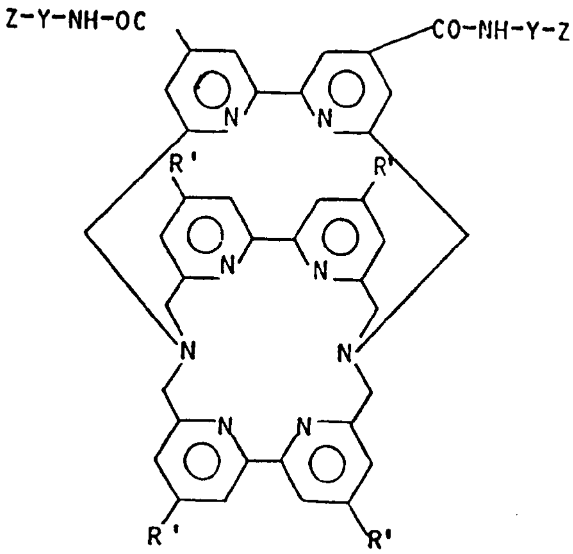

8

Selon ce procédé, on condense deux molécules du dérivé halogéné 6 avec une molécule du dérivé aminé 5. On opère avantageusement dans les mêmes conditions que dans la première étape du procédé A, c'est-à-dire que l'on réalise cette condensation dans un solvant organique anhydre, tel que l'acétonitrile en présence

d'une base telle qu'un carbonate d'ion alcalin, par exemple le carbonate de sodium ou le carbonate de lithium. On opère à la température de reflux du solvant. On obtient le composé macropolycyclique 7 qui est un intermédiaire-clé pour la synthèse des cryptates selon l'invention et fait partie également du cadre de l'invention. Ce complexe macropolycyclique est sous la forme d'un cryptate d'ion alcalin qui est avantageusement transformé en un cryptate de terre rare avant l'aminolyse selon le mode opératoire défini précédemment. Comme pour le procédé A, le cryptate d'ion alcalin 7 peut être, selon une variante de mise en oeuvre, décomplexé pour obtenir le composé macropolycyclique 7 correspondant ou cryptand libre, lequel est ensuite recomplexé en un cryptate de terre rare.

On procède ensuite à l'aminolyse du composé 7 selon le mode opératoire défini précédemment et on obtient le complexe macropolycyclique 8, c'est-à-dire un cryptate selon l'invention de formule I dans laquelle

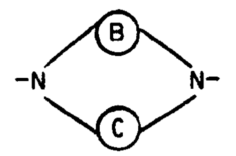

est le macrocycle bisbipyridine, Y, Z et R' sont tels que définis précédemment.

Le dérivé aminé 5 utilisé comme produit de départ dans le procédé B est obtenu à partir du dérivé halogéné 1 par transformation de celui-ci en le composé azido correspondant par réaction avec $NaN_3$ au reflux dans un solvant organique, tel que le tétrahydrofuranne, puis réduction du composé azido ainsi obtenu en présence d'un catalyseur de réduction, tel que le palladium sur charbon sous atmosphère d'hydrogène à la température ambiante.

PROCEDE C :

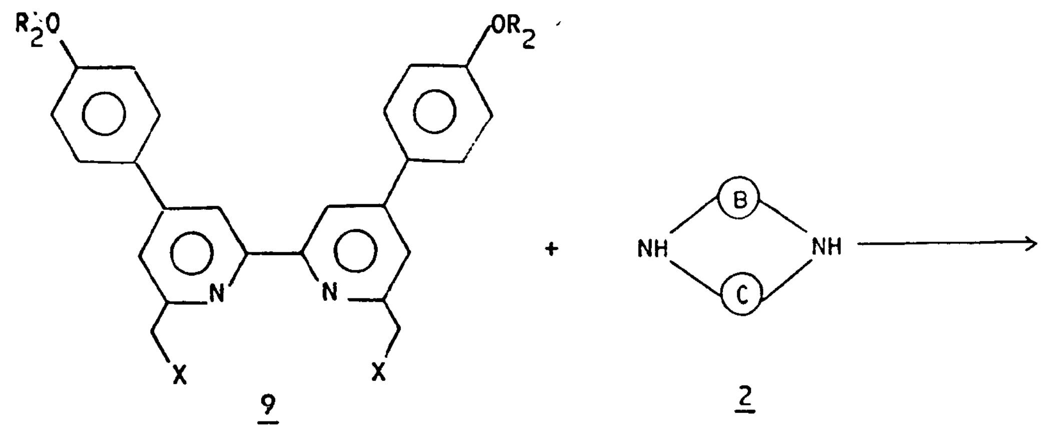

9 2

$R_2$ = H ou $CH_3$.

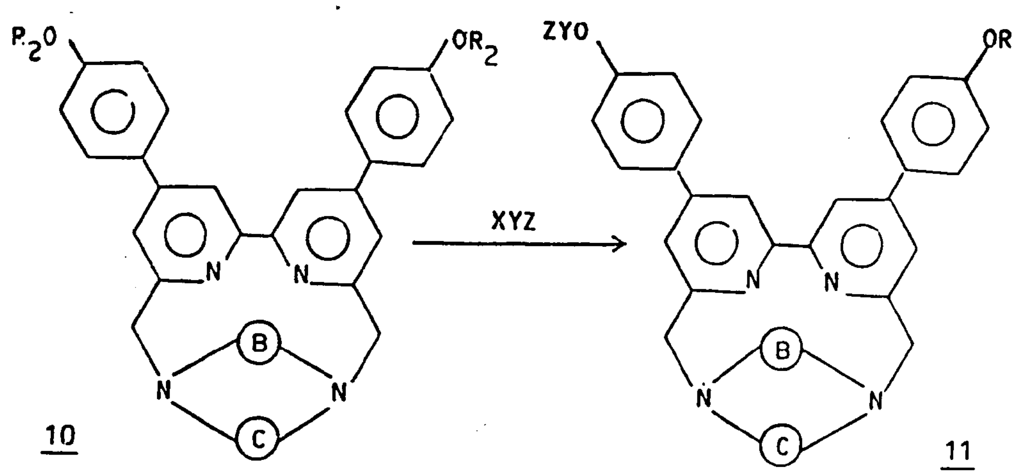


10 11

Selon ce mode opératoire, le dérivé halogéné de bipyridine convenablement substitué 9 est d'abord condensé avec le macrocycle 2. Cette réaction est avantageusement réalisée dans un solvant organique anhydre, tel que par exemple l'acétonitrile, en présence d'une base telle qu'un carbonate d'ion alcalin, par exemple le carbonate de sodium ou le carbonate de lithium. On obtient alors le composé macropolycyclique 10 sous la forme d'un cryptate d'ion alcalin. On opère de préférence à la température de reflux du solvant.

On procède ensuite à la substitution du composé macropolycyclique 10 en faisant réagir ce composé 10 avec un halogénure de formule XYZ, Z et Y étant tels que définis précédemment et X étant un ion halogénure.

Lorsque Z est un groupe cyano, celui-ci peut être transformé en un groupe amino par réduction à l'aide d'agents classiques de réduction.

Le composé 11 obtenu est ensuite transformé en un cryptate de terre rare par échange d'ions, par exemple selon le mode opératoire défini précédemment. Avantageusement, on dissout un halogénure de terre rare dans du méthanol et on y ajoute une solution du cryptate de l'ion alcalin dans du méthanol avec un faible volume de chloroforme sinécessaire. On chauffe le mélange au reflux sous atmosphère inerte et on suit la disparition du cryptate de l'ion alcalin par chromatographie sur couche mince.

Selon une variante de mise en oeuvre, le cryptate d'ion alcalin peut être décomplexé selon le mode opératoire défini précédemment (voir procédé A) et transformé en le cryptate de terre rare souhaité.

Lorsque la réaction est terminée, le cryptate de terre rare est isolé du milieu réactionnel selon des procédés classiques. Il se présente sous la forme d'un solide cristallin.

Le dérivé halogéné de bipyridine 9 mis en oeuvre dans le mode opératoire ci-dessus peut être obtenu selon le procédé représenté par le schéma réactionnel ci-aprés :

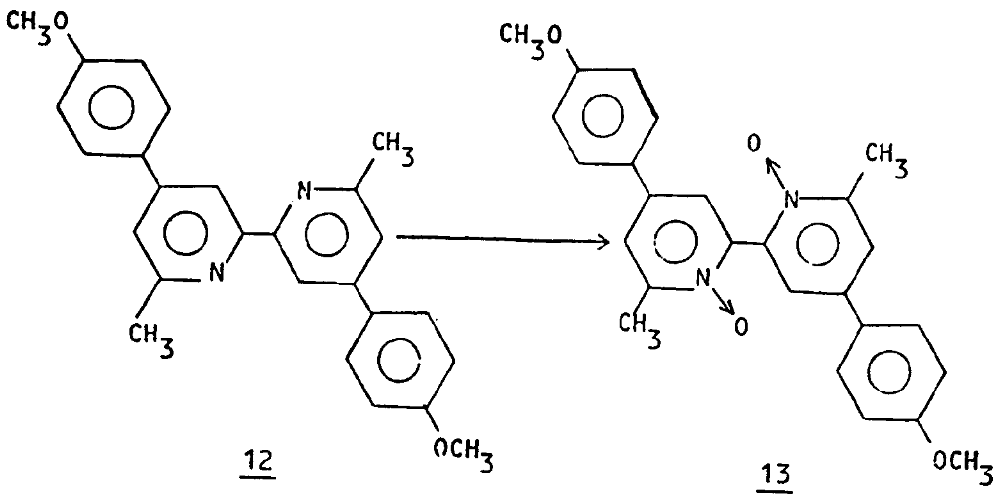

12 13

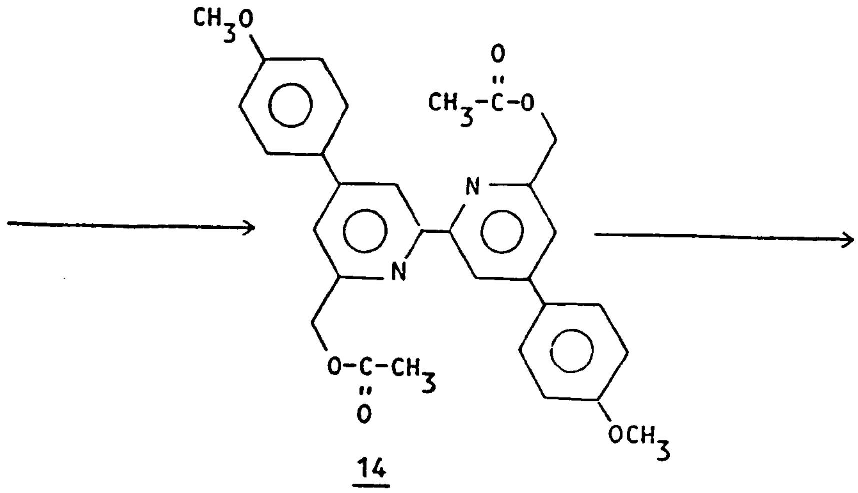

14

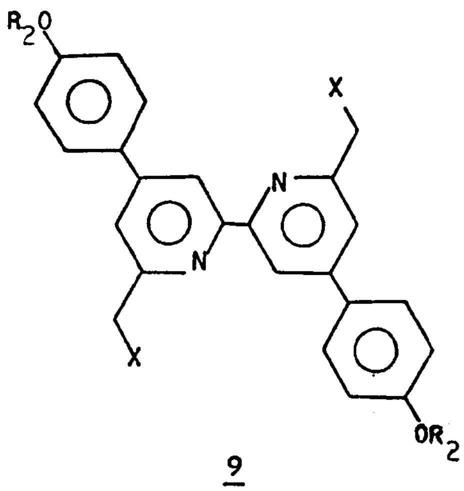

$R_2$ = H ou $CH_3$.

9

Le diméthyl-6,6′ di(p-méthoxyphényl)-4,4′ bipyridine-2,2′ de formule 12 peut être obtenu à partir du di(p-méthoxyphényl)-1,6 hexadiène-1,5 dione-3,4 selon la méthode de Kröhnke décrite dans SYNTHESIS (1976), 1.

Le composé 13 est obtenu par réaction du composé 12 avec de l'acide m-chloroperbenzoïque dans un solvant organique, tel que le chloroforme.

Le dérivé 14 est ensuite préparé par chauffage au reflux d'une suspension du composé 13 dans de l'anhydride acétique.

Par chauffage au reflux d'une solution du composé 14 avec un acide halogénohydrique en solution acétique, on obtient le composé 9 dans lequel $R_2$ est H ou $CH_3$, au moins l'un des radicaux $R_2$ étant l'hydrogène.

Le composé de formule 9 est un intermédiaire-clé pour l'obtention des composés de l'invention et constitue un autre objet de l'invention.

Les composés selon l'invention qui sont des cryptates stables de terres rares sont solubles dans l'eau et certains solvants organiques tels que le méthanol ou le DMSO.

Les composés de l'invention sont utiles comme marqueurs fluorescents des produits biologiques et conviennent donc comme réactifs de dosage dans les méthodes de détection et de détermination immunologique par fluorescence, aussi bien dans les dosages dits par compétition que ceux dits par excès.

L'invention va être maintenant décrite plus en détail dans les exemples illustratifs ci-après.

EXEMPLE 1 - Cryptate de terre rare de [(22)(p-$OCH_3$ p-$OCH_2CH_2NH_2$ diPhbpy)]

Formule II : R = $CH_3$, Y = $CH_2CH_2$, Z = $NH_2$ ,

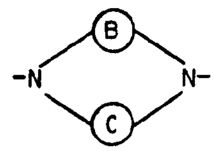

est le macrocycle (22) (procédé C).

A - Diméthyl-6,6′ di(p-méthoxyphényl)-4,4′ bipyridine-2,2′ (composé 12)

Un mélange de di(p-méthoxyphényl)-1,6 hexadiène-1,5 dione-3,4 (3,07 g, 9,5 mmol) préparé suivant la méthode de Kröhnke Synthesis (1976), 1, de chlorure de pyridinium-1 propane one-2 (3,27 g, 19,1 mmol) obtenu en mélangeant des quantités équimolaires de pyridine et de chloro-1 acétone et d'acétate d'ammonium (19 g) dans 95,5 ml de $CH_3OH$ est chauffé à reflux 38 h sous azote. Après retour du mélange réactionnel à la température ambiante, le précipité de couleur crème formé est filtré et lavé au $CH_3OH$. Le produit brut est utilisé sans purification supplémentaire. Il peut être cristallisé dans $CH_2Cl_2$/hexane.
Rendement : 70 %
C.C.M. : $R_f$ = 0,5 (silice, $CH_2Cl_2/CH_3OH$, 95/5)
S.M. 396 ($M^+$), 381 ($M^+$-$CH_3$), 365 ($M^+$-2$CH_3$), 353 ($M^+$-$CH_3$ -CO), 338 ($M^+$-2$CH_3$-CO), 198 ($M^+$/2).

B - Di (N-oxyde)-1,1′ diméthyl-6,6′ di(p-methoxyphényl)-4,4′ bipyridine-2,2′ (Composé 13)

A une solution de 12 (3,45 g, 8,7 mmol) dans 800 ml de $CHCl_3$, on additionne goutte à goutte, à 0°C, une solution d'acide m-chloroperbenzoïque (6 g, 34,8 mmol) dans 360 ml de $CHCl_3$. On laisse une nuit sous agitation à température ambiante. Le mélange est alors lavé avec une solution saturée de $NaHCO_3$ jusqu'à un pH alcalin. La phase organique est séparée et concentrée. L'ajout d'hexane provoque la précipitation du composé. Le précipité est filtré puis redissous dans un mélange $CH_2Cl_2/CH_3OH$ (90/10) et lavé avec une solution de soude 2N. La phase organique, qui contient le composé 13, est séparée et le solvant évaporé. Par ailleurs, le filtrat provenant de la précipitation est également lavé avec la solution de soude 2N, puis concentré et chromatographié sur une colonne d'alumine avec $CH_2Cl_2$ comme éluant.
Rendement : 80 %  F > 250°C
C.C.M : $R_f$ = 0,5 (alumine, $CH_2Cl_2/CH_3OH$, 95/5)
S.M. : 429 ($MH^+$), 411 ($M^+$-$H_2O$).

C - Diacétoxyméthyl-6,6′ di(p-méthoxyphényl)-4,4′ bipyridine-2,2′ (composé 14)

Une suspension de 13 (1,21 g, 2,82 mmol) dans 18 ml d'anhydride acétique est chauffée à reflux 1 h 30. La solution obtenue est concentrée. On ajoute 5 ml de $H_2O$ et 20 ml de $CH_2Cl_2$ au résidu pâteux et on basifie le milieu avec une solution aqueuse saturée de $NaHCO_3$. La phase organique est séparée, la phase aqueuse est extraite deux fois avec 30 ml de $CH_2Cl_2$. La phase organique résultante est séchée sur $Na_2SO_4$ et le solvant évaporé. Le produit brut est chromatographié sur une colonne d'alumine avec $CH_2Cl_2$ comme éluant.
Rendement : 90 %  F = 140-141°C
C.C.M. : $R_f$ = 0,9 (alumine, $CH_2Cl_2/CH_3OH$ , 95/5)
S.M. : 513 ($MH^+$), 469 ($M^+$-C(O)$CH_3$), 453 ($M^+$-$CH_3COOH$), 256 ($M^+$/2).

D - Dibromométhyl-6,6′ di(p-méthoxyphényl)-4,4′ bipyridine-2,2′ (composé 9a : $R_2$ = $CH_3$)

dibromométhyl-6,6′ (p-méthoxyphényl-4, p-hydroxyphényl-4′) bipyridine-2,2′ (composé 9b : l'un des radicaux $R_2$ = H, l'autre est $CH_3$).

Une solution de 14 (0,31 g, 0,60 mmol) dans 5 ml de HBr/AcOH 33 % est chauffée à reflux 12 à 13 h. On ajoute 20 ml de $H_2O$ et 60 ml de $CHCl_3$ à la solution à température ambiante. On lave avec une solution saturée de $NaHCO_3$ jusqu'à la neutralisation. La phase organique est séparée et la phase aqueuse extraite avec deux fois 20 ml de $CH_2Cl_2$. Le solvant est évaporé de la phase organique résultante et le résidu chromatographié sur une colonne d'alumine avec $CH_2Cl_2$, puis $CH_2Cl_2/CH_3OH$ (95/5) comme éluant.
Composé 9a ; $R_2$ = $CH_3$ ;
Rendement : 16 %
Décomposition : 210-215°C

C.C.M. : $R_f$ > 0,9 (alumine, $CH_2Cl_2/CH_3OH$, 95/5)
S.M. : 556,555,553 ($MH^+$), 556,554,552 ($M^+$), 475,473 ($M^+$-Br), 394 ($M^+$-2Br)
Composé 9b ; l'un des $R_2$ = H ;
Rendement : 35 %
C.C.M. $R_f$ 0,4 (alumine, $CH_2Cl_2/CH_3OH$, 95/5)
S.M. : 542,540,538 ($M^+$), 461,459 ($M^+$-Br), 380 ($M^+$-2Br);

E - Cryptate de sodium de [(22)(p-$OCH_3$ p-OH diPhbpy)] (composé 10)

Un mélange du macrocycle (22) (0,262 g, 1 mmol) et de $Na_2CO_3$ (1,05 g, 10 mmol) dans 600 ml de $CH_3CN$ anhydre est chauffé à reflux 30 min sous azote. On y ajoute alors une suspension du composé 9b (0,54 g, 1 mmol) dans 450 ml de $CH_3CN$ anhydre. Le mélange est chauffé 20 h à reflux sous azote. La solution obtenue est filtrée à chaud et le solvant évaporé. Le produit brut est chromatographié sur alumine avec $CH_2Cl_2/CH_3OH$ (95/5) comme éluant.
Rendement : 60-65 %
C.C.M. : $R_f$ = 0,6 (alumine, $CH_2Cl_2/CH_3OH$, 90/10)
S.M. : 663 ($M^+$), 640 ($M^+$-Na), 625 ($M^+$-Na-$CH_3$), 609 ($M^+$-Na-$OCH_3$), 595 ($M^+$-Na-$OCH_3$-OH).

F - Cryptate de sodium de [(22)(p-$OCH_3$ p-$OCH_2CN$ diPhbpy)] (composé 11a) : Z = CN ; Y = $CH_2$ ; R = $CH_3$.

A une solution de cryptate de sodium du composé 10 (0,042 g, 0,066 mmol) dans un volume minimum de $CH_3CN$, on ajoute un excès de NaH (en poudre) sous azote. On chauffe à reflux sous azote pendant 1 h. La solution est ramenée à température ambiante, avant l'addition de bromoacétonitrile (1,2 équivalent). Le mélange est agité à température ambiante, sous azote, toute la nuit. On ajoute 20 ml de $H_2O$ et 10 ml de $CH_2Cl_2$. On neutralise le milieu par une solution saturée de $NaHCO_3$. La phase organique est séparée et la phase aqueuse extraite deux fois avec 20 ml de $CH_2Cl_2$. La phase organique résultante est séchée sur $Na_2SO_4$, puis les solvants sont évaporés. Le produit brut est chromatographié sur une colonne d'alumine avec $CH_2Cl_2/CH_3OH$ (96/4) comme éluant.
Rendement : 75 %
C.C.M. $R_f$ = 0,5 (tache bleu-violet), (alumine, $CH_2Cl_2/CH_3OH$, 92,5/7,5)
S.M. : 702 ($M^+$), 679 ($M^+$-Na), 663 ($M^+$-Na-H-CH ), 654 ($MH^+$-Na-CN), 640 ($MH^+$-Na-$CH_2CN$).

G - Cryptate de sodium de [(22)(p-$OCH_3$ p-$OCH_2CH_2NH_2$ diPhbpy)] (composé 11b : Z = $NH_2$ ; Y = -$CH_2$-$CH_2$) ; R = $CH_3$)

A une suspension du 11a (0,078 g, 0,1 mmol) dans 8 ml de THF anhydre, on ajoute 2 ml de $B_2H_6$ (1M dans THF) et 5 ml de THF supplémentaire. On laisse sous agitation et à température ambiante pendant une nuit. On ajoute 10 ml de $CH_3OH$ et on agite 20 à 30 min. On évapore les solvants. On ajoute au résidu 20 ml d'un mélange $H_2O$/HCl concentré (1/1). On observe un précipité jaune et un dégagement gazeux. La dissolution a lieu peu à peu. On évapore les solvants et on traite le résidu obtenu par une solution aqueuse de NaOH (6N). L'amine 11b est extraite par 25 ml de $CH_2Cl_2$. Rendement quantitatif. La formation du composé est mise en évidence par $^1$H-RMN.

H - Réaction de complexation : Echange du sodium par un ion $Ln^{3+}$ (Eu, Tb)

On dissout $LnCl_3.xH_2O$ (0,1 mmol) dans 25 ml de $CH_3OH$, et on y ajoute une solution de cryptate de sodium obtenu précédemment (0,06 mmol) dans 4-8 ml de $CHCl_3$ (volume minimum). On chauffe le mélange à reflux sous atmosphère d'azote (6 heures à 4 jours selon le cryptate de sodium considéré). On suit la disparition du cryptate de sodium par C.C.M.(alumine, $CH_2Cl_2/CH_3OH$, 90/10). Lorsque la réaction est terminée, on filtre le mélange réactionnel si nécessaire et en évapore les solvants. On redissout le résidu dans 15-20 ml de $CH_3OH$ et on ajoute de l'éther jusqu'à persistance d'un trouble (5 ml à 25-30 ml). Le cryptate de terre rare formé cristallise ou précipite très rapidement ou après plusieurs heures selon la nature du cryptate. La formation du cryptate est mise en évidence par $^1$H-RMN et spectroscopie U.V. visible.

En suivant ce mode opératoire, on a obtenu respectivement le cryptate d'europium et le cryptate de terbium de formule 11, lesquels présentent les caractéristiques spectroscopiques ci-après :
Cryptate d'europium : UV/visible dans $CH_3OH$
280 nm maximum ; 292 nm épaulement ; 320 nm maximum.
Cryptate de terbium : UV/visible dans $H_2O$

282 nm; 310 nm épaulement.

EXEMPLE 2 : Cryptate de terre rare de [bpy.bpy.(p-$OCH_3$ p-$OCH_2CH_2NH_2$ diPhbpy)]

R = $CH_3$ ; Y = $CH_2$-$CH_2$-; Z = $NH_2$ ; R′ = H ;

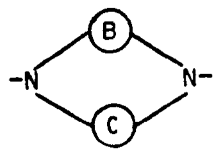

est le macrocycle bis-bipyridine (Procédé C).

A - Cryptate de sodium de [bpy.bpy.(p-$PCH_3$ p-OH diPhbpy)] (composé 10)

Un mélange de macrocycle bis-bipyridine (0.48 g, 1.21 mmol) et de $Na_2CO_3$ (1,16 g, 11 mmol) dans 480 ml de $CH_3CN$ anhydre est chauffé à reflux 30 min sous azote. On y ajoute une suspension de dibromométhyl-6,6′ (p-méthoxyphényl-4, p-hydroxyphényl-4′) bipyridine-2,2′ préparé précédemment (voir exemple 1 point D) (0,65 g, 1,20 mmol) dans 340 ml de $CH_3CN$. La solution résultante est chauffée 24 h à reflux, sous azote. Après retour à la température ambiante, le mélange réactionnel est filtré et le solvant évaporé. Le produit brut est alors chromatographié sur une colonne d'alumine avec $CH_2Cl_2/CH_3OH$ (98/2) comme éluant.
Rendement : 45 %
C.C.M. :
$R_f$ = 0,4 (alumine, $CH_2Cl_2/CH_3OH$, 90/10) tache violette (254 nm), verte (366 nm)
S. M. :
a) IC ($NH_3$) : 795 ($M^+$), 773 ($MH^+$-Na)

b) $FAB^+$ (thioglycérol) : 875 ($MBr^+$), 795 ($M^+$).

Le cryptate de sodium obtenu a été transformé en le cryptate de terbium en suivant le mode opératoire décrit à l'exemple 1H. Ce cryptate présente les caractéristiques spectroscopiques ci-après :

. UV/visible dans $H_2O$ :
244 nm (maximum) ; 303 nm (maximum)

. UV/visible dans $CH_3OH$ ;
244 nm (maximum) ; 301 nm (maximum); 318 nm (épaulement).

B - Cryptate de sodium de [bpy.bpy.(p-$OCH_3$ p-$OCH_2CN$ diPhbpy)]

A une solution de cryptate de sodium de 10 (0,21 g, 0,24 mmol) dans 12 ml de $CH_2Cl_2$ et 12 ml de $CH_3OH$, on ajoute une solution de NaOH dans $CH_3OH$ (1,5 équivalent dans 2 ml). Le mélange est chauffé 1 h 30 sous azote, à reflux. Puis, après retour à la température ambiante, le solvant est évaporé ; l'eau formée au cours de la réaction est éliminée par distillation azéotropique avec du toluène. Le produit ainsi obtenu est séché toute une nuit à la pompe à palette. Le composé orange vif obtenu est mis en solution dans 35 ml de THF (la solution est trouble). Dès l'ajout de bromoacétonitrile (30 µl, 1,8 équivalent), la solution devient limpide. On laisse réagir à température ambiante en agitant sous azote toute la nuit. On ajoute 20 ml de $H_2O$ et environ 10 ml de $CH_2Cl_2$. On neutralise le milieu par une solution saturée de $NaHCO_3$. La phase organique est séparée et la phase aqueuse extraite deux fois avec 20 ml de $CH_2Cl_2$. La phase organique résultante est séchée sur $Na_2SO_4$, puis les solvants sont évaporés. Le produit brut est chromatographié sur une colonne d'alumine avec $CH_2Cl_2/CH_3OH$ (95/5) comme éluant.
Rendement : 92 %
S.M. : $FAB^+$ 834 ($M^+$).

Le cryptate de sodium obtenu a été transformé en le cryptate de terbium correspondant selon le mode opératoire décrit à l'exemple 1. Ce cryptate présente les caractéristiques spectroscopiques ci-après :

. UV/visible dans $CH_3OH$ : 300 nm (maximum) ; 314 nm (épaulement).

C - Cryptate de terre rare de [bpy.bpy.(p-$OCH_3$ p-$OCH_2CH_2NH_2$ diPhbpy)]

En opérant selon le mode opératoire décrit au point G de l'exemple 1, on obtient le cryptate de terre rare du composé ci-dessus.

EXEMPLE 3 - Préparation du cryptate d'europium de [(bis-bpy) bpy-di(amidoéthylènamine)] (Procédé A)

Composé de formule I : Z = $NH_2$ ; Y = -$CH_2$-$CH_2$ ;

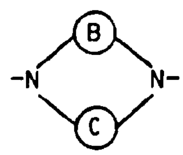

est le macrocycle bipyridine et R' est l'hydrogène.

A - Préparation du cryptate de sodium de [(bis-bpy) bpy diester]

Composé de formule 1 dans laquelle $R_1$ = $CH_3$.

Un mélange de 0,300 g (7,61.$10^{-4}$ mole) du macrocycle bis-bipyridine et de 1,10 g (10,4.$10^{-3}$ mole) de $Na_2CO_3$ est chauffé au reflux pendant 30 min. dans 450 ml de $CH_3CN$. Une solution de 0,350 g (7,64.$10^{-4}$ mole) de dibromométhyl-6,6' (p-diméthoxycarbonyl-4,4') bipyridine 2,2' (composé 1 ; X = Br) dans 375 ml de $CH_3CN$ est ensuite ajoutée sous forte agitation en 60 min. Le mélange réactionnel est encore agité 18 heures à reflux puis refroidi à température ambiante et filtré. Le solvant est éliminé sous vide à l'évaporateur rotatif. Le solide obtenu est dissous dans $CHCl_3$ et chromatographié sur colonne d'alumine (avec un peu de silice au sommet) par élution avec $CHCl_3/CH_3OH$ (98/2). Les solvants sont éliminés sous vide et 0,230 g (38 %) du complexe attendu sont obtenus. Celui-ci présente les caractéristiques physico-chimiques ci-après :
Fusion > 240°C
1H-RMN ($CD_3OD$) : 3,89 (S,8H, $CH_2$-bpy), 3,98 (S,4H, $CH_2$-bpy diE) ; 4,03 (S,6H, $COOCH_3$) ; 7,42 (d, J = 7,4 Hz, 4H-bpy) ; 7,92 (t-J = 7,4 Hz, 4H-bpy) ; 7,96 (d, J = 1,2 Hz, 2H-bpy diE) 8,10 (d, J = 7,4 Hz, 4H- bpy) ; 8,60 (d, J = 1,2 Hz, 2H-bpy diE) (diE = diester)
$^{13}$C-RMN($CDCl_3$) : 52,9, 59,5, 119,7, 120,4, 123,4, 124,1, 138,2, 139,7, 155,2, 155,7, 158,3, 160,4, 164,9
$C_{40}H_{34}N_8O_4NaBr\ 4H_2O$ (865,71) ;

Calculé (%) : C 55,49 ; H 4,89 ; N 12,94 ;
Trouvé (%) : C 55,44 ; H 4,33 ; N 13,10.

B - Aminolyse du cryptate ci-dessus

Le cryptate obtenu ci-dessus (0,100 g ; 1,26.$10^{-4}$ mole) est soumis à une aminolyse en l'ajoutant par portion à 4 ml d'éthylène diamine distillée sur KOH préalablement chauffée à 90°C. Le mélange réactionnel obtenu est encore agité pendant 1 heure puis refroidi à la température ambiante. L'éthylène diamine en excès est ensuite éliminée sous vide et on obtient une huile. Cette dernière est reprise dans 2 ml d'un mélange de $CHCl_3/CH_3OH$ (2/1) qui est à son tour éliminé sous vide. La purification est répétée 5 fois et on obtient alors le complexe attendu avec un rendement de 89 %.

C - Transformation du cryptate ci-dessus en le cryptate d'europium correspondant

En opérant selon le mode opératoire décrit au point H de l'exemple 1, on a obtenu le cryptate d'europium de [(bis-bpy)(bpy-di(amidoéthylènamine))] qui présente les caractéristiques spectroscopiques ci-après :
UV/visible dans $H_2O$
240 nm ; 303 nm (maxima)

EXEMPLE 4 - Préparation du cryptate d'europium de [(bis-bpy) (bpy-di(amidoéthlylènamine))] (Procédé A)

Composé de formule I : Z = $NH_2$ ; Y = $-CH_2-CH_2-$ ; R' = H

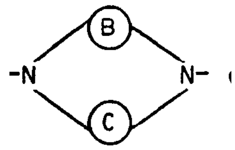

est le macrocycle bipyridine

A - Préparation du cryptate d'europium de [(bis-bpy) (bpy diester)]

Le cryptate de sodium obtenu au point A de l'exemple précédent (0,10 mmole) est dissous dans 8 ml de $CHCl_3$ et ajouté à une solution de $EuCl_3$, $6H_2O$ (0,15 mmole) dans 40 ml de $CH_3OH$. La solution est agitée au reflux, sous azote, jusqu'à la disparition du cryptate de sodium (CCM, oxyde d'aluminum, éluant ; $CH_2Cl_2$, $CH_3OH$ (90/10)). Le temps de réaction est de l'ordre de 48 heures. Le mélange réactionnel est refroidi à température ambiante et filtré s'il présente un trouble. Les solvants sont éliminés à l'évaporateur rotatif et le résidu est redissous dans 20-25 ml de $CH_3OH$. De l'éther éthylique est ajouté goutte-à-goutte jusqu'à la persistance d'un très léger trouble. Le cryptate d'europium cristallise ainsi à température ambiante. On élimine les solvants à la pipette et le cryptate est séché. Rendement : environ 40 % (première crystallisation). Le filtrat est concentré à secet l'opération est répétée.

Le produit obtenu présente les caractéristiques physico-chimiques ci-après :
1H-RMN ($D_2O$ réf = t-BuOH) : 10,51 (2H, S, CH(bpy diE)), 8,66 (2H,S, CH-bpy diE), 8,06 (4H, t, CH-bpy), 7,08 (4H, d, CH-bpy), 6,74 (4H, d, CH-bpy), 4,66 (6H, S, $OCH_3$), 0,9 (8H, S, $CH_2$-bpy), -0,78 (4H, S, $CH_2$-bpy diE)
$C_{40}H_{34}N_8O_4$ $EuCl_3$ NaCl, 9/2 $H_2O$ (1088,58)

| | | | | | | | |
|---|---|---|---|---|---|---|---|
| % calculé : | C | 44,13 ; | H | 3,98 ; | N | 10,29 |
| % trouvé : | C | 43,93 ; | H | 3,79 ; | N | 9,88 |
| | C | 44,06 ; | H | 3,70 ; | N | 10,03 |

B - Aminolyse du cryptate d'Europium

Le cryptate d'europium obtenu ci-dessus (40 mg) est ajouté par portion à 40 ml d'éthylène diamine préalablement distillé sur KOH. La solution limpide est agitée 3 heures sous azote à température ambiante. L'éthylène diamine est éliminée sous vide à l'évaporateur rotatif. Le résidu est repris dans quelques millilitres de $CH_2Cl_2$ / MeOH (2/1) et les solvants sont éliminés sous vide. L'opération est répétée 5 fois pour obtenir un composé parfaitement solide. Le cryptate obtenu est séché 24 heures sous le vide d'une pompe à palette.
Fusion = 168-170°C

Ce cryptate d'europium présente les mêmes caractéristiques que le cryptate d'europium obtenu à l'exemple précédent. Ces deux exemples montrent donc que l'on peut effectuer la complexation du cryptate de l'ion alcalin par un ion de terre rare avant ou après la substitution.

EXEMPLE 5 - Préparation du cryptate d'europium ou de terbium de [(22) bpy di(amidoéthylènamine)]

Composé de formule I =

I = 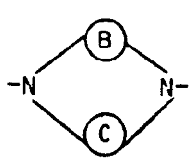

est le macrocycle (22) ; Z = $NH_2$ ; Y = $-CH_2-CH_2-$

A - Préparation du cryptate de sodium de [(22) bpy diester]

Un mélange de 0,114 g ($4,37.10^{-4}$ mole) du macrocycle (22) et de 0,460 g ($4,34.10^{-3}$ mole) de $Na_2CO_3$ est chauffé 30 min. au reflux dans 130 ml de $CH_3CN$. Une solution de 0,200 g ($4,37.10^{-4}$ mole) du composé 1 dans 220 ml de $CH_3CN$ est ensuite ajoutée, sous forte agitation, en 60 min. Le mélange réactionnel est encore agité 15 heures au reflux puis refroidi à température ambiante et filtré. Le solvant est éliminé sous vide à l'évaporateur rotatif. Le solide obtenu est dissous dans du $CH_2Cl_2$ et purifié par chromatographie sur colonne d'alumine) avec un peu de silice à son sommet). Une impureté est d'abord éluée au $CH_2Cl_2$. Le macrobicycle formé est ensuite élué par $CH_2Cl_2/CH_3OH$ (98/2). Les solvants sont éliminés sous vide et 0,135 g (61 %) du macrobicycle sont obtenus.

B - Préparation du cryptate d'europium ou de terbium du [(22) bpy diester]

En procédant selon le mode opératoire décrit à l'exemple 1H, on a obtenu respectivement les cryptates d'europium et de terbium de [(22) bpy diester], lesquels présentent les caractéristiques spectroscopiques ci-après :

– cryptate d'europium : UV/visible dans $CH_3OH$
242 nm et 324 nm (maxima)
– cryptate de terbium : UV/visible dans $CH_3OH$
242 nm et 325 nm (maxima)

C - Préparation du cryptate d'europium du [(22) bpy di(amidoéthylèneamine)]

Le cryptate d'europium obtenu ci-dessus (0,100 g, $1,43.10^{-4}$ mole) est ajouté par portion à 5ml d'éthylène diamine, distillée sur KOH, préalablement chauffée à 90°C. Le mélange réactionnel est encore agité 1 heure, puis refroidi à température ambiante. L'éthylène diamine en excès est éliminée sous vide. Une huile est obtenue. Cette dernière est reprise dans 2 ml de $CHCl_3$ qui est à son tour éliminé sous vide. La purification est répétée 3 fois et 93 mg (90 %) du cryptate d'europium sont obtenus. Ce cryptate présente les caractéristiques spectroscopiques ci-après :
UV/visible dans $H_2O$.
240 et 315 nm (maxima)

De la même façon, on peut soumettre à aminolyse le cryptate de terbium obtenu au point B.

EXEMPLE 6 - Préparation du cryptate de sodium de [(bpy dicarbométhoxy)$_3$] (Procédé B)

Formule 7 R′ = R″ = $OCH_3$ ;

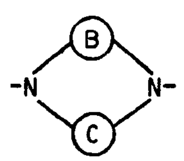

est le macrocycle bis-bipyridine

A - Synthèse du dicarbométhoxy-4,4′ diazidométhyl-6,6′ bipyridine 2,2′

Un mélange de dicarbométhoxy-4,4′ dibromométhyl-6,6′ bipyridine-2,2′ (0,50 g, $1,10.10^{-3}$ mole) et de $NaN_3$ (1,0 g, $15,4.10^{-3}$ mole) est chauffé à reflux pendant 36 heures dans 15 ml de THF. Le mélange réactionnel est refroidi à température ambiante filtré sur célite et concentré à sec. Le résidu est dissous dans du $CHCl_3$ et chromatographié sur colonne de silice par élution avec du $CHCl_3$.

L'évaporation du solvant conduit à 0,40 g (95 %) du composé attendu qui présente les caractéristiques physico-chimiques ci-après :
F = 168-170°C
1H-RMN ($CDCl_3$) : 4,02 (S, 6H, $COOCH_3$) ; 4,63 (S, 4H, $CH_2$-H3) ; 7,94 (d, J = 1,3 Hz, 2H) ; 8,96 (d, J = 1,3 Hz, 2H) ;
$^{13}$C-RMN ($CDCl_3$) : 52,7, 55,1 ; 120,0 ; 121,5 ; 139,7 156,0, 156,8 ; 165,3 ;
IR (KBr) : 1715 $cm^{-1}$ (ester), 2090 $cm^{-1}$ (azide)

MS 383 ($(MH)^+$)
$C_6H_{14}N_8O_4$ (382,34)

% calculé : C 50,27 ; H 3,69 ; N 29,31
% trouvé : C 50,37 ; H 3,51 ; N 27,69.

B - Synthèse du dicarbométhoxy-4,4′ diaminométhyl-6,6′ bipyridine 2,2′ (composé 5)

Un mélange du composé obtenu selon A (0,126 g, $3,30.10^{-4}$ mole) et de 12,6 mg de 10 % de Pd/C dans 38 ml de $CH_2Cl_2/CH_3OH$ (2/1) est agité à température ambiante sous atmosphère d'hydrogène pendant 12 heures.

Le mélange réactionnel est filtré sur célite et l'évaporation des solvants conduit à 0,100 g (92 %) du composé attendu qui est utilisé sans purification dans l'étape ci-après.

C - Synthèse du cryptate de sodium du [bpy-dicarbométhoxy]$_3$ (composé 7)

Un mélange de 6 (R′ = $OCH_3$ ; X = Br) (0,280 g, $6,11.10^{-4}$ mole) et de $Na_2CO_3$ (0,65 g, $6,13.10^{-3}$ mole) est chauffé à reflux dans 500 ml de $CH_3CN$. Puis, 0,100 g ($3,03.10^{-4}$ mole) de 5 sont ajoutés et le mélange réactionnel est encore agité à reflux pendant 48 heures. Le mélange réactionnel est refroidi à température ambiante et filtré. Le solvant est éliminé sous vide. Le résidu est repris dans $CHCl_3$ et chromatographié sur colonne d'alumine (avec un peu de silice au sommet) en éluant avec $CHCl_3$/MeOH (98/2). L'évaporation des solvants conduit à 0,132 g (40 %) du macrobicycle 7 (R″ = R′ = $OCH_3$).
F. > 250°C
1H-RMN ($CDCl_3$) : 4,02 (S, 18H, $COOCH_3$) ; 4,08 (S, 12H, $CH_2$) ; 7,96 (d, J = 1,2 Hz, 6H) ; 8,49 (d, J = 1,2 Hz, 6H) ;
$^{13}C$-RMN ($CDCl_3$) : 53,2 ; 59,2 ; 120,0 ; 123,8 ; 139,9 ; 155,6 ; 159,9 ; 164,8.
$C_{48}H_{42}N_8O_{12}$, NaBr, $3H_2O$ (1079,84)

% calculé : C 53,39 ; H 4,48 ; N 10,38
% trouvé : C 53,40 ; H 4,40 ; N 9,75.

Le cryptate obtenu ci-dessus peut être soumis à une aminolyse avec de l'éthylène diamine selon le mode opératoire décrit dans les exemples précédents. On obtient généralement un mélange de cryptates mono-, di- à hexa-substitués par le groupe CO-NH-$CH_2$-$CH_2$-$NH_2$ ; Ces composés peuvent être séparés par des moyens classiques.

EXEMPLE 7 : Préparation du cryptate d'europium du [bis(bpy dicarboxybutoxy), bpy-diamidoéthylène amine]

Formule I = Y = -$CH_2$-$CH_2$- ; Z = $NH_2$ ;

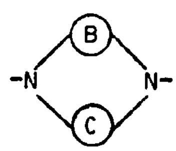

est le macrocycle bis-bipyridine ; R′ = t-Bu (tertiobutyle ou B[t])

A - Préparation du cryptate de sodium du [bis(bpy dicarboxybutoxy), bpy dicarboxyméthoxy]

En opérant selon le mode opératoire décrit à l'exemple 6 et en utilisant le composé 5 obtenu au point B de cet exemple et le composé 6 (R′ = COO(t-$C_4H_9$) ; X = Br), on a obtenu le cryptate de sodium de formule 7 (R′ = COO(t-$C_4H_9$) ; R″ = $COOCH_3$) qui présente les caractéristiques physico-chimiques ci-après :
F. > 220°C
1H-RMN ($CDCl_3$) : 1,60 (S, 36H, COOBu[t]) ; 3,99 ; 4,02 ; 4,06 ; (3S, 18H, $CH_2$-bpy di COO Me + $CH_2$-bpy di

COO $Bu^t$, + $-COOCH_3$) ; 7,81 (S, 4H, bpy di COO $Bu^t$) ; 7,95 (S, 2H, bpy di COO Me) ; 8,36 (S, 4H, bpy di COO $Bu^t$) ; 8,45 (S, 2H, bpy di COO Me)
$^{13}C$-RMN ($CDCl_3$) : 28,0 ; 53,1 ; 59,1 ; 83,4 ; 119,8 ; 119,9 ; 123,4 ; 123,7 ; 139,8 ; 141,7 ;
$C_{60}H_{66}N_8O_{12}$, NaBr, $3H_2O$ (1248,18)

% calculé : C 57,73 ; H 5,81 ; N 8,98

% trouvé : C 57,99 ; H 5,31 ; N 9,03.

Ce cryptate de sodium a été ensuite transformé en le cryptate d'europium correspondant selon le mode opératoire décrit précédemment.

Celui-ci a été ensuite soumis à aminolyse avec de l'éthylènediamine.

Selon une variante, on peut d'abord soumettre à aminolyse le composé 7 ci-dessus puis transformer le cryptate de sodium obtenu en le cryptate d'europium correspondant en suivant les modes opératoires décrits précédemment.

APPLICATION DES COMPOSES A TITRE DE MARQUEURS FLUORESCENTS

Comme indiqué précédemment, les composés de l'invention conviennent comme agents pour le marquage de substances biologiques qui est réalisé par couplage du composé de l'invention par liaison covalente avec la substance biologique à doser ou à détecter. Ce couplage peut être réalisé selon les procédés classiques de couplage utilisés dans ce domaine, tels que par exemple les procédés au SMCC/SPDP ou au carbodiimide (voir l'ouvrage Laboratory Techniques in Biochemistry and Molecular Biology - Practise and Theory on Enzym- and Immunoassays P. TIJSSEN, Elsevier 1 ed. 1985).

Les cryptates selon l'invention conviennent comme marqueurs fluorescents dans tous les types de procédés de détermination ou de détection de substances biologiques et notamment dans les méthodes de dosage dites par compétition ou par excès, en phase homogène ou en phase hétérogène, bien connus de l'homme de l'art et décrits notamment par LANDON, Ann. Clin. Biochem. 1981, 18, p. 253 et SOINI Clin. Chemi. 25, 353, 1979. Ils peuvent être également utilisés dans le procédé homogène de détection et/ou de détermination par luminescence décrit dans la demande internationale WO 87/00927.

Les substances biologiques à détecter ou à déterminer peuvent être par exemple les anticorps, les antigènes, les toxines, les enzymes, les protéines, les hormones, les récepteurs d'hormones, les stéroïdes, l'avidine, la biotine, la streptavidine, les microorganismes, les haptènes, les acides nucléiques, les fragments d'ADN et d'ARN, les lipides, les glucides, etc. Des exemples de telles substances sont cités dans la description de la demande de brevet EP 17 908 incorporée dans la présente description à titre de référence.

L'utilisation des composés de l'invention comme marqueurs fluorescents est illustrée par les cinq essais ci-après :

Dans les essais A, D et E ci-après, on a utilisé les réactifs SPDP, et sulfo-SMCC qui sont respectivement : "N-succinimidyl 3-(2- pyridylthio) propionate" et "sulfosuccinimidyl 4-(N-maleimido méthyl) cyclohexane-1-carboxylate".

ESSAI A : formation d'un conjugué anticorps-cryptate par SMCC/SPDP

1) Activation du cryptate d'europium obtenu selon l'exemple 3

A 500 µl d'une solution de ce cryptate à 1,5 mg/ml dans un tampon phosphate 20 mM pH 7, on ajoute 50 µl de sulfo SMCC 13,1 mg/ml dans le même tampon.

On incube ensuite pendant 30 min. à température ambiante et on agite au vortex pendant 5 min. On centrifuge la solution pour éliminer le précipité. On purifie par passage sur une colonne échangeuse d'ions Pharmacia Mono Q en formant un gradient entre :

Tampon A : phosphate 20 mM pH 7 + 10 % DMF

Tampon B : phosphate 20 mM pH 7 + 10 % DMF + 1 molaire NaCl

Le cryptate est détecté par mesure de son absorption à 307 nm ($\varepsilon$ = 25000 $M^{-1}$ $cm^{-1}$ à 307 nm).

– le cryptate activé sort dans le volume mort

– l'excès de sulfo SMCC est élué dans le gradient.

2) Activation de l'anticorps

L'anticorps monoclonal $E_1$ utilisé est l'anticorps anti-prolactine contenu dans les trousses pour les dosages immunoradiométriques de la prolactine fabriquées par la compagnie ORIS INDUSTRIE et connues sous la dénomination "ELSA PROL".

A 1,2 ml d'une solution d'anticorps $E_1$ à 14,8 mg/ml dans un tampon phosphate 50 mM, 150 mM NaCl pH 7,1, on ajoute 30 µl d'une solution de SPDP 30 mM dans l'éthanol absolu.

On incube 30 min à température ambiante en agitant.

La solution est passée sur une colonne de gel G25 pharmacia équilibrée avec le même tampon phosphate ($\varepsilon$= 210 000 $M^{-1}$ $cm^{-1}$ à 280 nm).

On récupère la fraction éluée dans le volume mort.

On ajoute 60 µl d'une solution à 400 mM de DTT (dithiothréitrol) dans le tampon phosphate.

On incube 15 min à température ambiante sous agitation et on récupère la fraction sortant dans le volume mort après passage sur une colonne G25 équilibrée dans le tampon phosphate.

3) Couplage entre l'anticorps activé et le cryptate activé

A 60 µl d'anticorps activé à 2,6 mg/ml dans un tampon phosphate, on ajoute 50 µl de cryptate à 200 µmole/l.

On incube 12 heures à température ambiante et on centrifuge. Le surnageant est dialysé contre un tampon phosphate 50mM pH 7,4.

ESSAI B : Formation d'un conjugué anticorps-cryptate par carbodiimide

1) Succinilation de l'anticorps

On ajoute en trois fois 83 µl d'une solution à 50 mg/ml d'anhydride succinique dans 1/3 DMSO 2/3 borate 100 mM pH 9 à 440 µl d'anticorps $E_1$ à 9,4 mg/ml dans du borate 100 mM pH 9 plongée dans la glace fondante.

Le pH est maintenu à 9 par la soude. On laisse réagir 30 min à 0°C.

2) Purification

Elle est réalisée par chromatographie haute pression sur colonne de gel filtration TSK 3000 SW, l'éluant étant un tampon phosphate 50 mM pH 7,4. Débit 1 ml/min.

On récupère l'anticorps succinilé sur 3 ml. On concentre pour obtenir 370 µl.

3) Couplage anticorps succinilé/cryptate

Solution 1 : on mélange 25,5 mg de carbodiimide dans 1335 µl de tampon phosphate 50 mM pH 7,4

Solution 2 : 50 mg/ml de sulfo N-hydroxy succinimide dans le même tampon (14,7 mg dans 222,7 µl)

Solution 3 : 0,96 mg/ml de cryptate selon l'exemple 3 dans le même tampon.

On fait réagir 1 heure à température ambiante :

– 175 µl de solution d'anticorps succinilé
– 480 µl solution 3
– 72,5 µl solution 1
– 10 µl solution 2

Après dialyse exhaustive contre tampon phosphate 50 mM pH 7,4, le produit est concentré sur cône Amicon. On obtient un conjugué à 0,86 mg/ml.

Le produit est congelé dans le tampon avec 1 mg/ml de serum albumine humaine (HSA).

RESULTATS : DOSAGE DE LA PROLACTINE

On utilise les réactifs de la trousse de dosage de prolactine ELSA-PROL.

Dans chaque tube contenant la phase solide ELSA, on ajoute :

– 100 µl de standard
– 300 µl de conjugué $E_1$ -cryptate obtenu selon les essais A ou B en tampon phosphate 50 mM pH 7,4 et HSA 1 mg/ml.

On incube 3 heures à 37°C.

On lave la phase solide avec 2 fois 3 ml d'$H_2O$.

On ajoute 400 µl de tampon contenant 5 mMolaire en sodium dodecyl sulfate (SDS).

On transfère 300 µl de cette solution pour la mesure de la fluorescence sur un appareil ARCUS LKB.

Les résultats obtenus sont portés sur la figure 1 annexée. Sur cette figure, on a porté en ordonnées l'intensité de la fluorescence et en abscisses, la quantité de prolactine en µU/ml.

Ces résultats montrent que les cryptates selon l'invention conviennent comme marqueurs fluorescents dans un dosage immunologique.

Dans l'essai C ci-après, on illustre l'utilisation des cryptates selon l'invention dans le procédé homogène selon la demande internationale WO 87/00927.

ESSAI C : Dosage de la prolactine

I - Dosage homogène de la prolactine

A - Fixation d'iode sur l'anticorps

On marque l'anticorps monoclonal anti-prolactine $G_2$ avec le réactif de Wood [décrit dans synthèse Anal. Biochem. 69, 339-349 (1975)].

2,93 mg du réactif de Wood sont dissous dans 40 µl de soude 0,5 N.

On ajoute 400 µl de tampon carbonate 200 mM, pH 9,3.

On ajoute à cette solution 200 µl d'anticorps $G_2$ à 10 mg/ml dans un tampon carbonate 100 mM, pH 9,3 et on incube pendant 17 heures à 30°C.

On purifie sur une colonne Pharmacia PD10 avec un tampon carbonate 100 mM, pH 9,3.

Les fractions éluées dans le volume mort sont recueillies ; Le conjugué est caractérisé par un rapport de densité optique à 320 et 280 nm $\frac{\text{Do } 320}{\text{Do } 280} \simeq 1{,}4$ à 1,5

Sa concentration est estimée en admettant que 90 % des anticorps sont récupérés. Le conjugué est aliquoté et conservé à - 20°C.

B - Dosage de la prolactine (courbe A)

On utilise des standards prolactine à 0,15, 30, 60 et 150 ng/ml. Le dosage s'effectue dans des barettes de 12 puits EFLAB. n° catalogue 96 02 107.

On ajoute successivement :

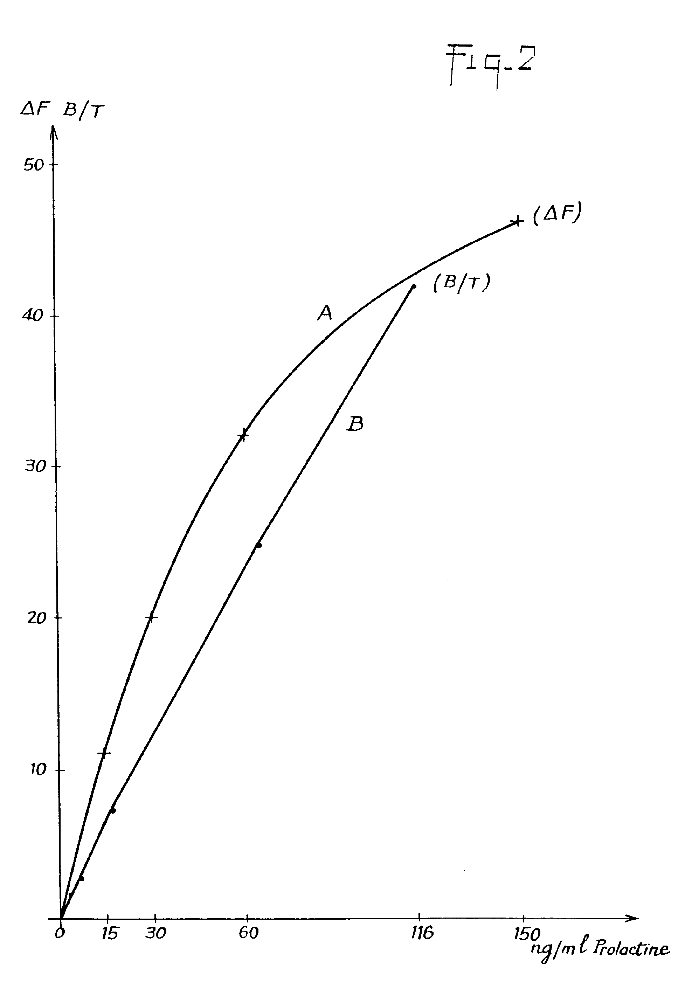

- 100 µl de standard
- 100 µl d'anticorps $G_2$ Wood à 5 µg/ml
- 100 µl de conjugué $E_1$ de l'essai A à 0,375 ug/ml ;
- on incube 15 minutes à température ambiante,
- on effectue la lecture sur l'appareil ARCUS,
- on trace une courbe standard en portant la valeur Δ F en fonction de la valeur des standards (figure 2)

$$\text{avec } \Delta F = \frac{\text{Fluorescence du standard mesuré} - \text{Fluorescence du standard 0}}{\text{Fluorescence du standard 0}}$$

I- Dosage de la prolactine par radioimmunométrie (courbe B)

On utilise la trousse ELSA-PRL (Compagnie ORIS).

- dans le tube ELSA, on ajoute :
- 300 µl de traceur radioactif,
- 100 µl de standard,
- après agitation, on incube 3 heures à 37°C,
- les tubes sont lavés 2 fois à l'eau,
- les tubes sont comptés sur un compteur ,
- on porte la valeur B/T pour chaque standard (figure 2) avec

B = radioactivité comptée sur le standard

T = radioactivité comptée pour 300 µl du traceur radioactif.

Les résultats obtenus (voir figure 2) montrent que les cryptates selon l'invention conviennent comme marqueurs dans le procédé homogène selon la demande internationale WO 87/00927. D'autre part, on peut noter que grâce aux cryptates selon l'invention, ce procédé homogène est particulièrement avantageux par rapport au procédé de dosage utilisant un élément radioactif, lequel nécessite un temps d'incubation beaucoup plus

long, des étapes de lavage et la manipulation d'éléments radioactifs.

ESSAI D - Formation d'un conjugué ADN-cryptate par SMCC/SPDP

1/ Fonctionnalisation de l'ADN en ADN-$NH_2$

Un nucléotide modifié, le 5-amino(12)dUTP (produit commercialisé par CALBIOCHEM) est introduit dans un ADN donné par nick translation selon le mode opératoire préconisé par CALBIOCHEM. L'ADN aminé est ensuite purifié par trois lavages successifs sur un filtre "Centricon" (commercialisé par AMICON) avec du tampon phosphate 10mM pH 7,4.

2/ Couplage de l'ADN-$NH_2$ avec du SPDP

55 µl d'une solution contenant 9 µg d'ADN-$NH_2$ (mesuré par UV) dans du tampon borate 0.1 M pH 9,0 sont ajoutés à 5 µl d'une solution de SPDP 20 MM. La réaction dure 4 heures à 45°C. Le SPDP n'ayant pas réagi est éliminé par trois lavages sur "Centricon" avec un tampon phosphate 50 mM pH 7,0.

3/ Déprotection de l'ADN-SPDP et couplage au cryptate

90 µl d'une solution de 6 µg (estimé par densité optique) d'ADN-SPDP dans du tampon phosphate 50 mM pH 7,0 sont traités juste avant couplage par 10 µl d'une solution 10 mM de DTT dans le même tampon. La déprotection dure 20 minutes à température ambiante. Le DTT inhibiteur du couplage suivant est éliminé par trois lavages sur "Centricon" avec le tampon phosphate précédent. La solution d'ADN-SH est concentrée sur"Centricon"jusqu'à un volume final de 90 µl.

90 µl d'une solution d'ADN-SH sont mis en présence de 50 µl du cryptate d'europium de l'exemple 3 activé au SMCC selon le point 1 de l'essai A (14 mM) dans du tampon phosphate 20 mM pH 7,0 contenant 10 % de DMF. Le couplage se fait à 30°C pendant 1 heure. Le conjugué ADN-cryptate est purifié par trois précipitations successives à l'éthanol. Le taux de couplage est évalué par mesure de l'absorption à 260 nm et de la fluorescence à 620 nm. 4/ Une sonde ADN ainsi couplée à un cryptate peut être utilisée pour la détection et le dosage d'une séquence d'acide nucléique complémentaire selon une des stratégies décrites dans la revue de J. MATTHEWS et al. dans Analytical Biochemistry 169, 1-25(1988) intitulée "Analytical strategies for the use of DNA probes".

ESSAI E - Formation d'un conjugué covalent streptavidine-cryptate

1/ Couplage streptavidine - SPDP

20 µl d'une solution 20 mM de SPDP dans un tampon carbonate 0,5 M pH 9,5 sont ajoutés à 180 µl d'une solution contenant 1 mg de streptavidine dans le même tampon.

La réaction dure une heure à température ambiante. Le SPDP en excés est éliminé par quatre lavages successifs sur "Centricon"avec du tampon phosphate 0,1 M, pH 7,4.

2/ Déprotection de la streptvaidine-SPDP et couplage au cryptate

A 360 µl d'une solution de streptavidine - SPDP (≈1 mg) dans du phosphate 0,1 M pH 7,4 on rajoute 40 µl de DTT 100 mM dans le même tampon.

La réaction dure 15 minutes à température ambiante sous agitation puis le mélange réactionnel est lavé trois fois sur "Centricon" avec le tampon précédent.

La streptavidine-SH (≈ 1 mg) dans 625 µl de tampon phosphate 0,1 M pH 7,4 est couplée à 43 µg du cryptate d'europium de l'exemple 3 activé au SMCC selon le point 1 de l'essai A dans 175 µl de tampon phosphate 20 mM pH 7 avec 10 % de DMF. Le couplage dure 2 h 30 à température ambiante. Le cryptate-SMCC n'ayant pas réagi est éliminé par quatre lavages sur "Centricon" avec du tampon phosphate 0,1 M pH 7,4.

Le taux de couplage est estimé par mesure de l'absorption à 280 nm et de la fluorescence à 620 nm.

Le conjugué obtenu est purifié par gelfiltration sur colonne TSKG3000 SW avec un tampon NaCl 1 M, TFA 0,1 %. La détection se fait par mesure de l'absorption à 280 nm et de la fluorescence à 620 nm.

3/ un tel conjugué streptavidine-cryptate peut être utilisé comme marqueur fluorescent de molécules biotinylées (acides nucléiques, oligonucléotides, anticorps, antigènes, haptènes ou autres substances biologiques) reconnaissant elles-mêmes des biomolécules pour lesquelles elles ont une grande affinité dans un

mélange selon un procédé de dosage ou de détection classique.

## Revendications

1. Cryptates de terres rares, caractérisés en ce qu'ils sont constitués d'au moins un sel de terre rare complexé par un composé macropolycyclique répondant à l'une des formules I ou II ci-après :

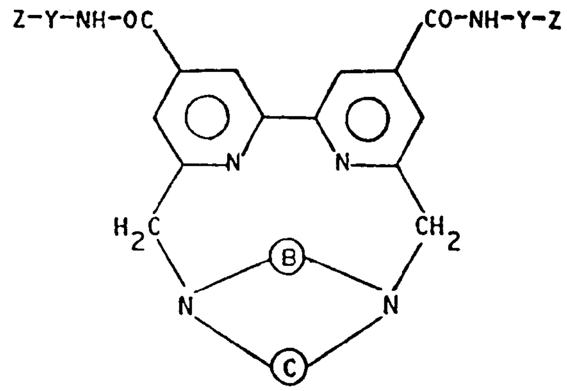

I

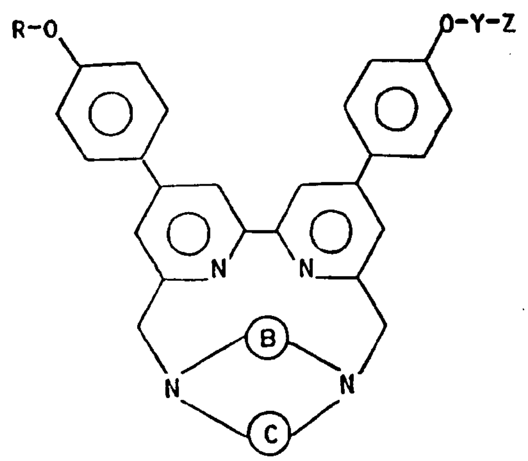

II

dans lesquels :

– le cycle de formule

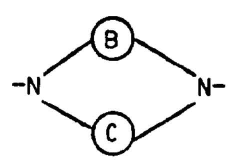

est l'un des cycles suivants :

1) 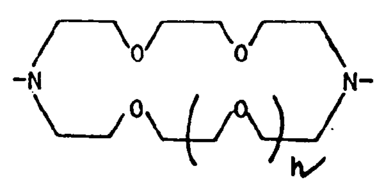

n = 0 ou 1

macrocycle $[N_2O_4]$ ou cycle (22)

macrocycle [$N_2O_3$] ou cycle (21)

2) 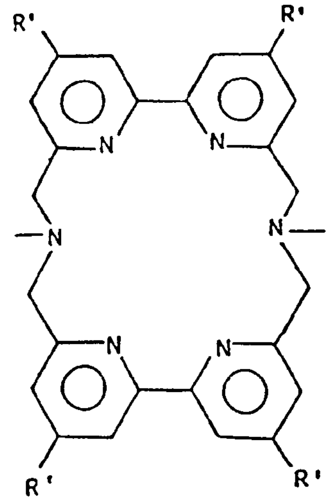

macrocycle bis-bipyridone

– Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou étant éventuellement interrompus par un ou plusieurs hétéroatomes, tels que l'oxygène, l'azote, le soufre ou le phosphore, parmi les groupes cycloalkylène en $C_5$ à $C_8$ ou parmi les groupes arylène en $C_6$ à $C_{14}$ lesdits groupes alkylène cycloalkylène ou arylène étant éventuellement substitués par des groupes alkyle, aryle ou sulfonate;

– Z est un groupe fonctionnel susceptible de se lier de façon covalente avec une substance biologique ;

– R est un groupe méthyle ou représente le groupe -Y-Z ;

– R′ est l'hydrogène ou un groupe -COOR″ dans lequel R″ est un groupe alkyle en $C_1$ à $C_{10}$ et représente de préférence le groupe méthyle, éthyle ou tertiobutyle ou bien R′ est un groupe-CO-NH-Y-Z.

2. Cryptates selon la revendication 1 répondant à la formule I, caractérisés en ce que Y est $-CH_2-CH_2-$ et Z est $-NH_2$

3. Cryptates selon l'une des revendications 1 ou 2, caractérisés en ce que

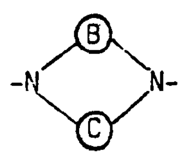

est le macrocycle bis-bipyridine

4. Cryptates selon la revendication 1, répondant à la formule II, caractérisés en ce que Y est $-CH_2-$, Z est CN et R est le groupe méthyle.

5. Cryptates selon la revendication 1, répondant à la formule II, caractérisés en ce que Y est $-CH_2-CH_2-$, Z est NH et R est le groupe méthyle.

6. Procédé pour l'obtention des cryptates de formule I selon la revendication 1, caractérisé en ce qu'il consiste à soumettre le composé de formule :

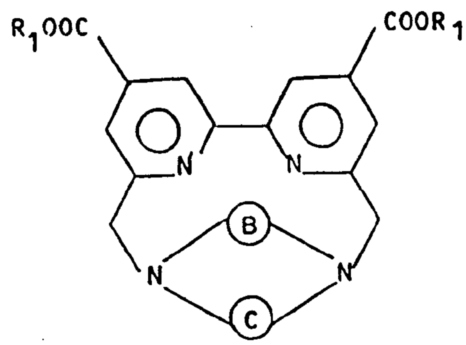

dans laquelle $R_1$ est un groupe alkylène en $C_1$-$C_{10}$, à une aminolyse par réaction avec l'amine de formule $NH_2$-Y-Z dans laquelle Y et Z sont tels que définis dans la revendication 1, la réaction étant effectuée sous atmosphère d'azote à la température ambiante, et à transformer le composé obtenu en un cryptate de terre rare par échange d'ions.

7. Procédé selon la revendication 6, pour l'obtention des composés de formule I dans laquelle

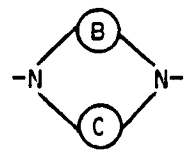

est le macrocycle bis-bipyridine, caractérisé en ce que le composé de formule 3 est obtenu par réaction d'une molécule de

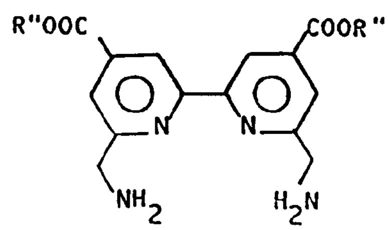

5

dans laquelle R″ est tel que défini dans la revendication 1 avec deux molécules du composé de formule

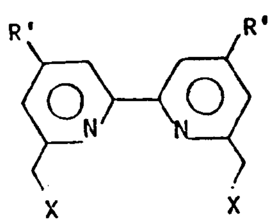

6

dans laquelle R′ est tel que défini dans la revendication 1 et X est un ion halogénure, la réaction étant réalisée dans un solvant organique anhydre à la température de reflux du solvant.

8. Procédé pour l'obtention des cryptates de formule II, selon la revendication 1, caractérisé en ce qu'il consiste à faire réagir en solution dans un solvant organique anhydre et à la température de reflux dudit solvant, le composé halogéné 9

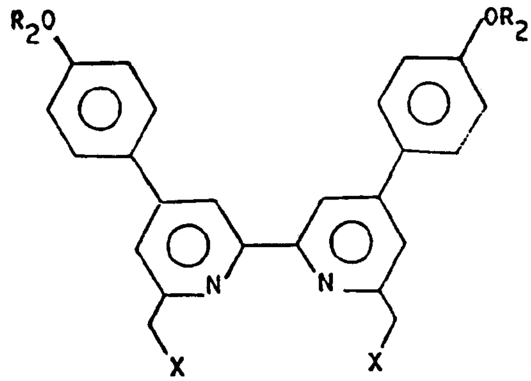

9

$R_2$ = H ou $CH_3$
avec le macrocycle

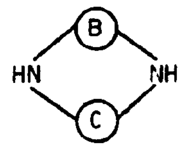

pour former le complexe macropolycyclique 10

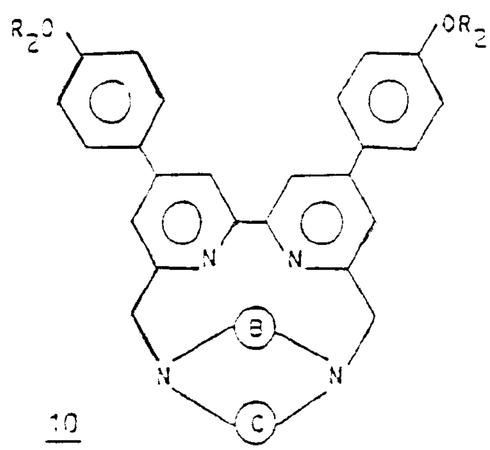

10

qui est ensuite traité avec le composé halogéné de formule XYZ, dans laquelle Y et Z sont tels que définis dans la revendication 1 et X est un ion halogénure.

9. A titre d'intermédiaires de synthèse, les complexes macropolycycliques de formule

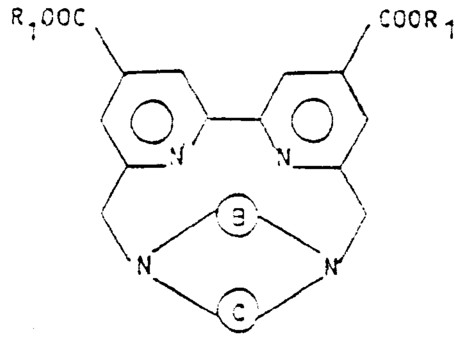

3

dans laquelle $R_1$ et

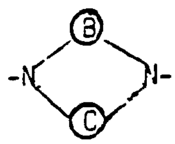

sont respectivement tels que définis dans les revendications 1 et 6.

10. A titre d'intermédiaires de synthèse, les composés de formule :

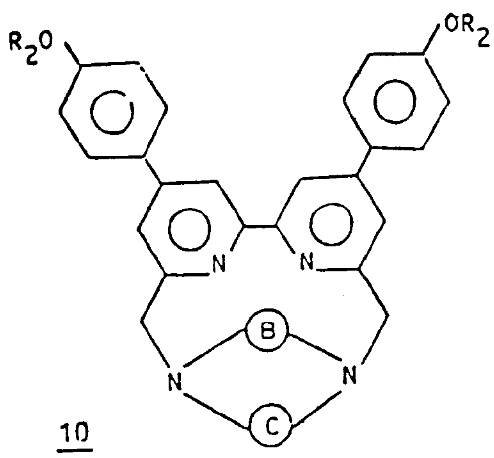

10

dans laquelle

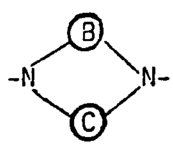

est tel que défini dans la revendication 1 et $R_2$ est H ou $CH_3$.

11. Intermédiaires selon l'une des revendications 9 ou 10, caractérisés en ce qu'ils sont sous la forme de cryptants libres.

12. Intermédiaires selon l'une des revendications 9 ou 10, caractérisés en ce qu'ils sont sous la forme de cryptates d'ion alcalin ou de cryptate de terre rare.

13. Application des cryptates selon l'une quelconque des revendications 1 à 5, à titre de marqueurs fluorescents.

14. Application des cryptates selon l'une quelconque des revendications 1 à 5, à titre de marqueurs fluorescents dans les procédés de détection et/ou de détermination de substances biologiques et notamment dans les procédés homogénes par luminescence.

## Patentansprüche

1. Kryptate Seltener Erden, dadurch gekennzeichnet, daß sie gebildet sind durch mindestens ein Salz einer Seltenen Erde, das mittels einer makropolyzyklischen Verbindung einer der nachstehenden Formeln I oder II

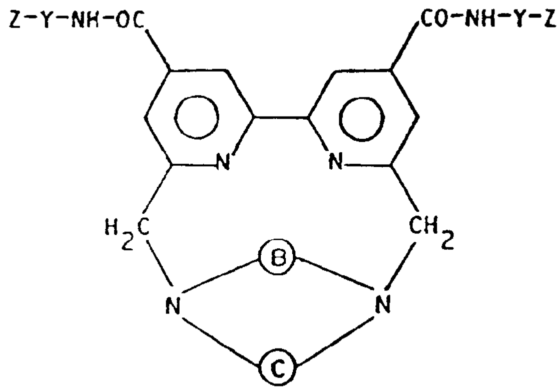

I

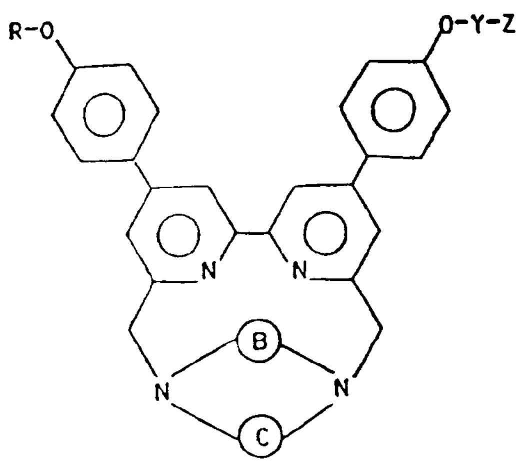

II

worin

– der Ring der Formel

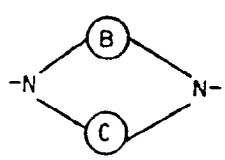

einen der folgenden Ringe darstellt:

1) 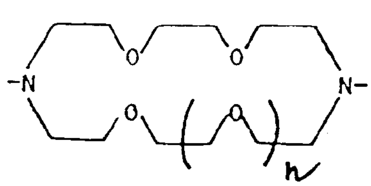

n = 0 oder 1
Makroring [$N_2O_4$] oder Ring (22)
Makroring [$N_2O_3$] oder Ring (21)

2)

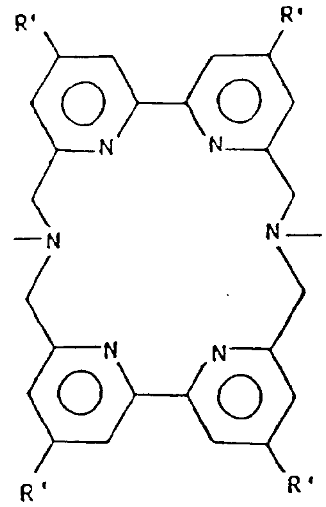

Bis-bipyridin-Makroring
– Y eine Raumgruppe oder eine Raumbrücke ist, bestehend aus einem bivalenten organischen Rest, ausgewählt aus linearen oder verzweigten $C_1$-$C_{20}$-Alkylengruppen, die gegebenenfalls eine oder mehrere Doppelbindungen enthalten und/oder gegebenenfalls durch ein oder mehrere Heteroatome, wie Sauerstoff, Stickstoff, Schwefel oder Phosphor, unterbrochen sind, aus $C_5$-$C_8$-Cycloalkylengruppen oder aus $C_6$-$C_{14}$-Arylengruppen, wobei die Alkylen-, Cycloalkylen- oder Arylengruppen gegebenenfalls durch Alkyl-, Aryl- oder Sulfonatgruppen substituiert sind;
– Z eine funktionelle Gruppe ist, die sich auf kovalente Weise mit einer biologischen Substanz binden kann;
– R Methyl ist oder die Gruppe -Y-Z darstellt;
– R′ Wasserstoff oder eine Gruppe -COOR″ ist, in welcher R″ eine $C_1$-$C_{10}$-Alkylgruppe ist und vorzugsweise Methyl, Ethyl oder tert.Butyl darstellt, oder aber R′ eine Gruppe -CO-NH-Y-Z ist, komplexiert ist.

2. Kryptate nach Anspruch 1 der Formel I, dadurch gekennzeichnet, daß Y für $-CH_2-CH_2-$ steht und Z $NH_2$-ist.

3. Kryptate nach einem der Ansprüche 1 oder 2, dadurch gekennzeichnet, daß

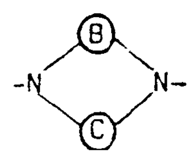

der Bis-bipyridin-makroring ist.

4. Kryptate nach Anspruch 1 der Formel II, dadurch gekennzeichnet, daß Y für $-CH_2-$ steht, Z CN ist und R Methyl ist.

5. Kryptate nach Anspruch 1 der Formel II, dadurch gekennzeichnet, daß Y für $-CH_2-CH_2-$ steht, Z $NH_2$ ist und R Methyl ist.

6. Verfahren zur Herstellung der Kryptate der Formel 1 nach Anspruch 1, dadurch gekennzeichnet, daß es darin besteht, daß die Verbindung der Formel

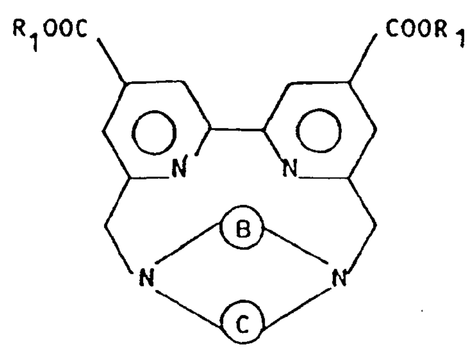

3

worin $R_1$ eine $C_1$-$C_{10}$-Alkylengruppe darstellt, einer Aminolyse durch Reaktion mit dem Amin der Formel $NH_2$-Y-Z, worin Y und Z wie in Anspruch 1 definiert sind, unterzogen wird, wobei die Reaktion unter Stickstoffatmosphäre bei Umgebungstemperatur ausgeführt wird, und daß die erhaltene Verbindung durch Ionenaustausch in ein Kryptat einer Seltenen Erde übergeführt wird.

7. Verfahren nach Anspruch 6 zur Herstellung der Verbindungen der Formel 1, worin

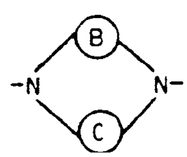

der Bis-pyridinmakroring ist, dadurch gekennzeichnet, daß die Verbindung der Formel 3 erhalten wird durch Umsetzung eines Moleküls von

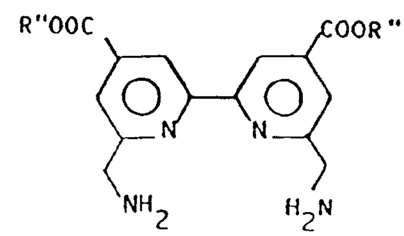

5

worin R″ wie in Anspruch 1 definiert ist, mit zwei Molekülen der Verbindung der Formel

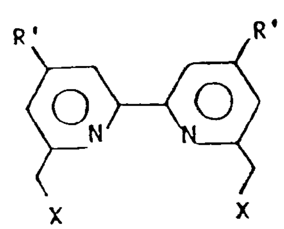

6

worin R′ wie in Anspruch 1 definiert ist und X ein Halogenidion darstellt, welche Reaktion in einem wasserfreien organischen Lösungsmittel bei der Rückflußtemperatur des Lösungsmittels durchgeführt wird.

8. Verfahren zur Herstellung der Kryptate der Formel II nach Anspruch 1, dadurch gekennzeichnet, daß es darin besteht, daß die Halogenverbindung 9

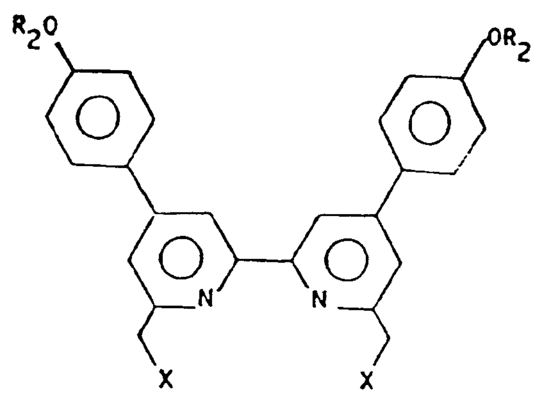

9

$R_2$ = H oder $CH_3$
in Lösung in einem wasserfreien organischen Lösungsmittel und bei der Rückflußtemperatur des Lösungsmittels mit dem Makrozyklus

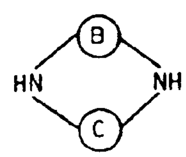

reagieren gelassen wird, un dem makropolyzycyklischen Komplex 10

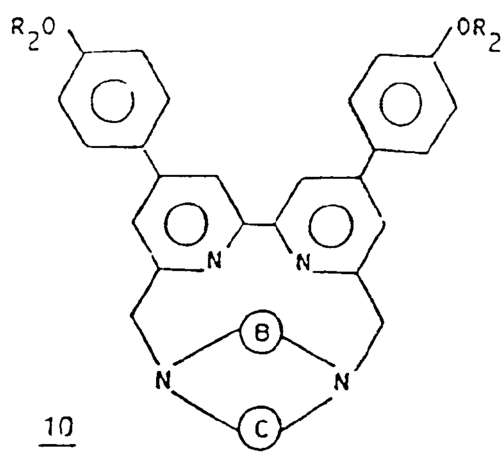

10

zu bilden, der danach mit der Halogenverbindung der Formel XYZ behandelt wird, worin Y und Z wie in Anspruch 1 definiert sind und X ein Halogenidion ist.

9. Als Synthesezwischenprodukte die makropolyzyklischen Komplexe der Formel

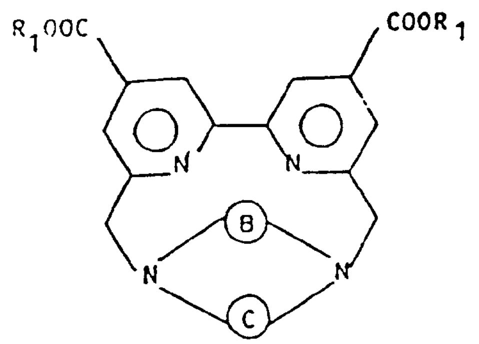

worin $R_1$ und

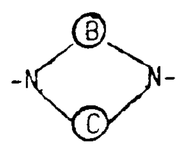

wie in den Ansprüchen 1 bzw. 6 definiert sind.

10. Als Synthesezwischenprodukte die Verbindungen der Formel

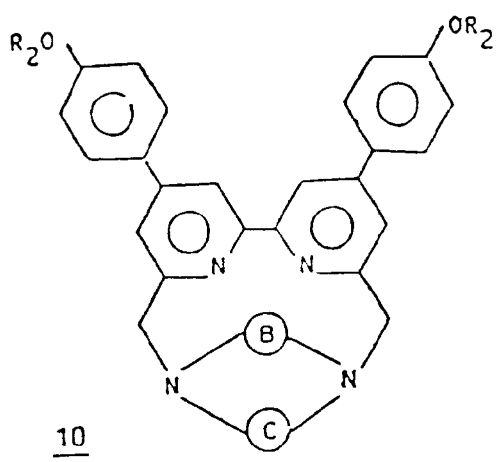

10

worin

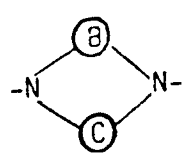

wie in Anspruch 1 definiert ist und $R_2$ für H oder $CH_3$ steht.

11. Zwischenprodukte nach einem der Ansprüche 9 oder 10, dadurch gekennzeichnet, daß sie in Form von freien Kryptanden vorliegen.

12. Zwischenprodukte nach einem der Ansprüche 9 oder 10, dadurch gekennzeichnet, daß sie in Form von Alkalikryptaten oder von Kryptaten Seltener Erden vorliegen.

13. Verwendung der Kryptate nach einem der Ansprüche 1 bis 5 als fluoreszierende Tracer.

14. Verwendung der Kryptate nach einem der Ansprüche 1 bis 5 als fluoreszierende Tracer in Verfahren zum Nachweis und/oder zur Bestimmung von biologischen Substanzen, insbesondere in homogenen Verfahren mittels Lumineszenz.

## Claims

1. Rare earth cryptates, characterized in that they consist of at least one rare earth salt complexed by a macropolycyclic compound having one of formulae I or II below:

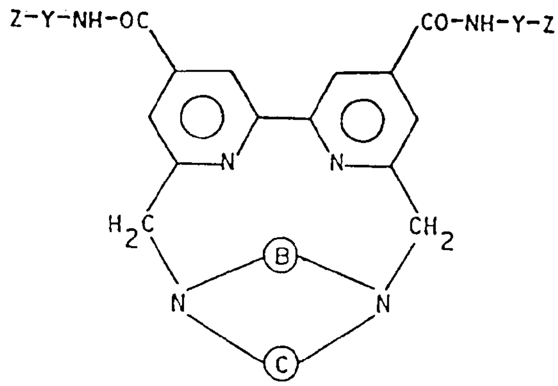

I

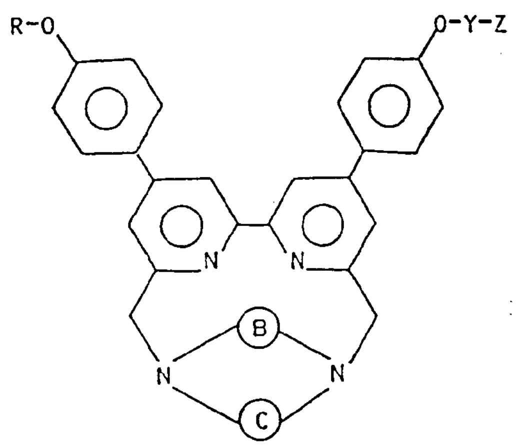

II

in which:

– the ring of the formula

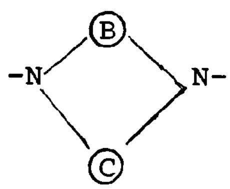

is one of the following rings:

1) 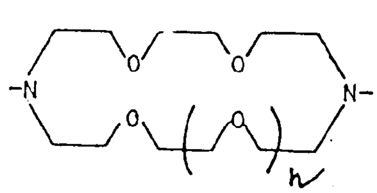

n = 0 or 1

$[N_2O_4]$ macrocycle or (22) ring

$[N_2O_3]$ macrocycle or (23) ring

2) 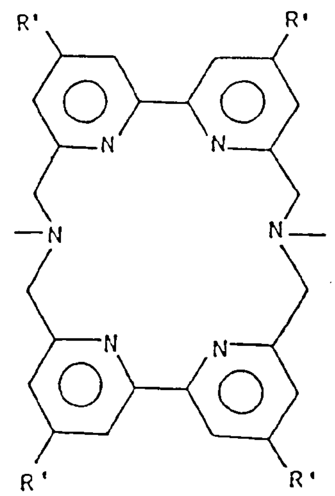

bis-bipyridine macrocycle

– Y is a spacer group or arm consisting of a divalent organic radical selected from linear or branched $C_1$ to $C_{20}$ alkylene groups, which may or may not contain one or more double bonds and/or may or may not be interrupted by one or more heteroatoms such as oxygen, nitrogen, sulfur or phosphorus, from $C_5$ to $C_8$ cycloalkylene groups or from $C_6$ to $C_{14}$ arylene groups, said alkylene, cycloalkylene or arylene groups being unsubstituted or substituted by alkyl, aryl or sulfonate groups;

– Z is a functional group capable of bonding covalently with a biological substance;

– R is a methyl group or represents the group -Y-Z; and

– R′ is hydrogen or a group -COOR″, in which R″ is a $C_1$ to $C_{10}$ alkyl group and preferably represents the methyl, ethyl or tert-butyl group, or R′ is a group -CO-NH-Y-Z.

2. Cryptates according to claim 1 of formula I, characterized in that Y is $-CH_2-CH_2-$ and Z is $-NH_2-$.

3. Cryptates according to one of claims 1 or 2, characterized in that

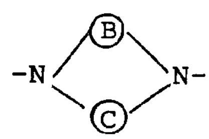

is the bis-bipyridine macrocycle.

4. Cryptates according to claim 1 of formula II, characterized in that Y is $-CH_2-$, Z is CN and R is the methyl group.

5. Cryptates according to claim 1 of formula II, characterized in that Y is $-CH_2-CH_2-$, Z is NH and R is the methyl group.

6. Method of obtaining the cryptates of formula I according to claim 1, characterized in that it consists in subjecting the compound of the formula:

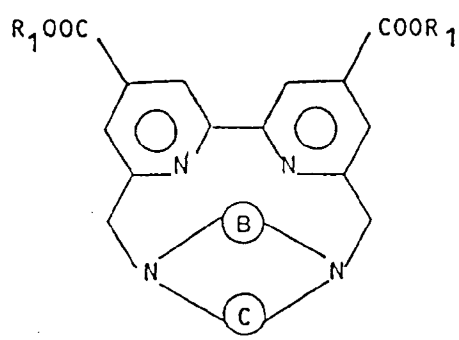

3

in which $R_1$ is a $C_1$-$C_{10}$ alkylene group, to aminolysis by reaction with the amine of the formula $NH_2$-Y-Z, in which Y and Z are as defined in claim 1, the reaction being carried out under a nitrogen atmosphere at room temperature, and in converting the resulting compound to a rare earth cryptate by ion exchange.

7. Method according to claim 6, of obtaining the compounds of formula I in which

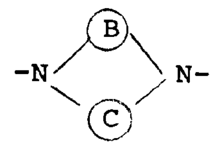

is the bis-bipyridine macrocycle, characterized in that the compound of formula 3 is obtained by reacting one molecule of

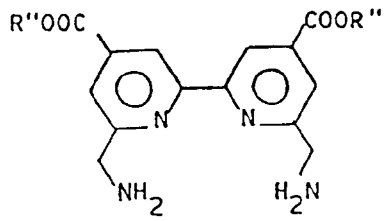

5

in which R″ is as defined in claim 1, with two molecules of the compound of the formula

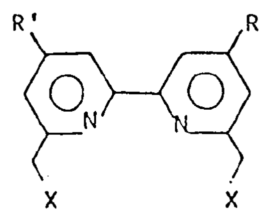

6

in which R′ is as defined in claim 1 and X is a halide ion, the reaction being carried out in an anhydrous organic solvent at the reflux temperature of the solvent.

8. Method of obtaining the cryptates of formula II, according to claim 1, characterized in that it consists in reacting the halogenated compound 9:

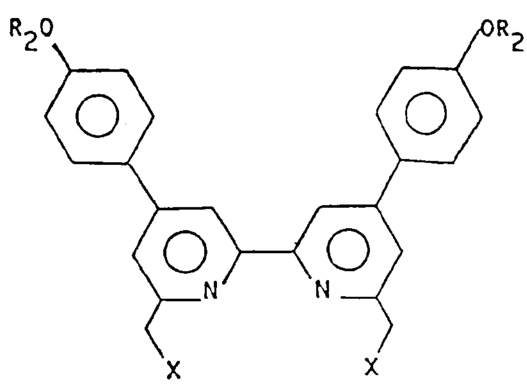

9

$R_2$ = H or $CH_3$
with the macrocycle

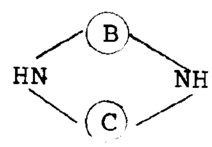

,

in solution in an anhydrous organic solvent and at the reflux temperature of said solvent, to form the macropolycyclic complex 10:

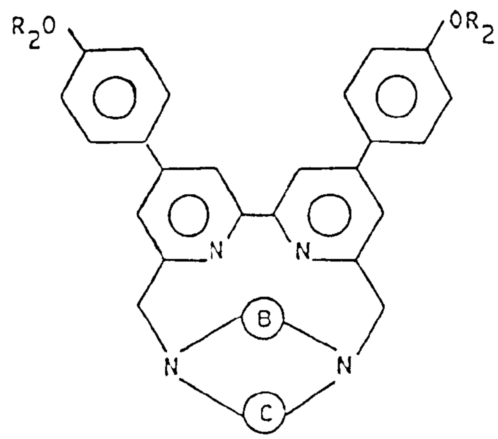

10

which is then treated with the halogenated compound of the formula XYZ, in which Y and Z are as defined in claim 1 and X is a halide ion.

9. As synthesis intermediates, the macropolycyclic complexes of the formula

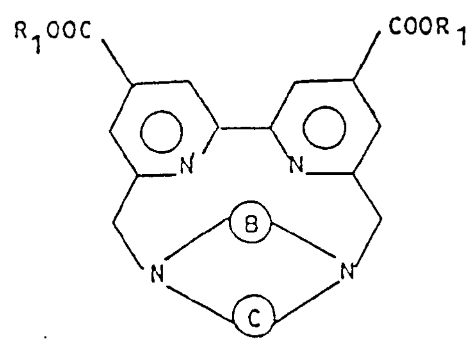

3

in which $R_1$ and

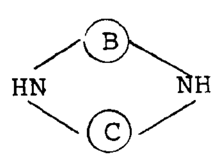

are as defined in claims 1 and 6 respectively.

10. As synthesis intermediates, the compounds of the formula

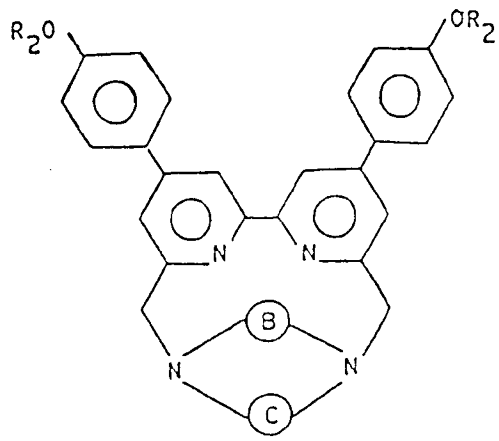

10

in which

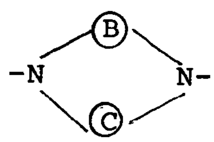

is as defined in claim 1 and $R_2$ is H or $CH_3$.

11. Intermediates according to one of claims 9 or 10, characterized in that they are in the form of free cryptants.

12. Intermediates according to one of claims 9 or 10, characterized in that they are in the form of alkali metal ion cryptates or rare earth cryptate.

13. Application of the cryptates according to any one of claims 1 to 5 as fluorescent tracers.

14. Application of the cryptates according to any one of claims 1 to 5 as fluorescent tracers in methods for the detection and/or determination of biological substances, and especially in homogeneous luminescence methods.

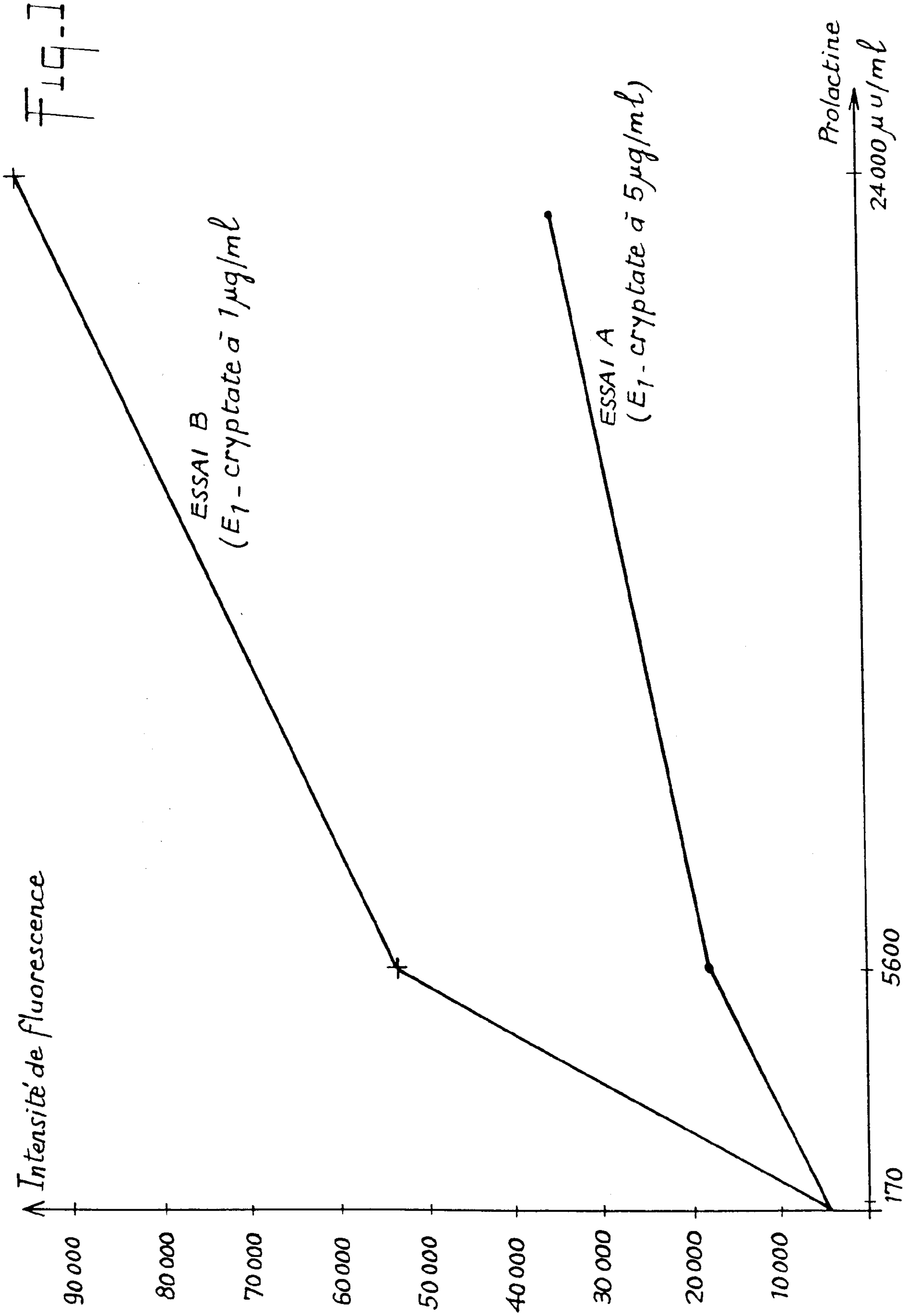
Intensité de fluorescence
90 000
80 000
70 000
60 000
50 000
40 000
30 000
20 000
10 000
ESSAI B
(E1 - cryptate à 1 µg/ml
ESSAI A
(E1 - cryptate à 5 µg/ml)
Prolactine
µU/ml
170
5600
24 000

Fig-1

Fig-2